# EUROPEAN PATENT APPLICATION

(11) **EP 2 599 851 A2**
(43) Date of publication of application: **05.06.2013**
(21) Application number: 11812803.2
(22) Date of filing: 29.07.2011
(51) Int. Cl.: C09K 11/06, C07F 15/00, H01L 51/50, C07D 209/82, H01L 27/32, H05B 33/14

(54) **ORGANIC ELECTROLUMINESCENT DEVICE EMPLOYING ORGANIC LIGHT EMITTING COMPOUND AS LIGHT EMITTING MATERIAL**

(30) Priority: 29.07.2011 KR 20110075593; 30.07.2010 KR 20100074290
(71) Applicant: Rohm And Haas Electronic Materials Korea Ltd., Chungcheongnam-do 331-980 (KR)
(72) Inventor: KIM, Chi Sik, Seoul 121-789 (KR); LEE, Soo Yong, Cheonan-si Chungcheongnam-do 331-930 (KR); KIM, Young Gil, Anyang-si Gyeonggi-do 431-075 (KR); LEE, Hyo-Jung, Seoul 153-784 (KR); LEE, Su Hyun, Suwon-si Gyeonggi-do 440-709 (KR); KIM, Hyun, Anyang-si Gyeonggi-do 431-080 (KR); CHO, Young Jun, Cheonan-si Chungcheongnam-do 331-930 (KR); CHANG, Hoon, Daejon 302-120 (KR); LEE, Kyung Joo, Daejeon 305-762 (KR); KIM, Bong-Ok, Seoul 135-090 (KR); KIM, Sung-Min, Seoul 158-766 (KR)
(74) Representative: Buckley, Guy Julian
(86) International application number: PCT/KR2011/005618
(87) International publication number: WO 2012/015274

(57) **Abstract**

Provided is an organic electroluminescent device that exhibits an efficient host-dopant energy transfer mechanism, and thus, expresses a certain high-efficiency electroluminescent performance, based on improved electron density distribution. The organic electroluminescent device also overcomes low initial efficiency and short operation life property, and secures high-performance electroluminescent performance with high efficiency and long life property for each color.

## Description

### FIELD OF THE INVENTION

The present invention relates to an organic electroluminescent device, and more particularly to an organic electroluminescent device where an organic layer is interposed between an anode and a cathode on a substrate, the organic layer including an electroluminescent layer containing one or more dopant compounds represented by Chemical Formula 1 below and one or more host compounds represented by Chemical Formulas 2 to 5 below.

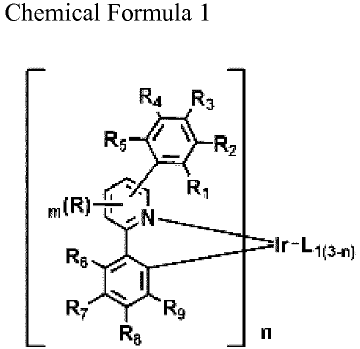

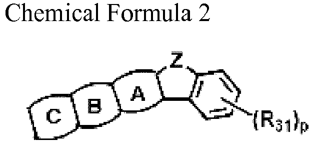

(Cz-L₂)ₐ-M Chemical Formula 3

(Cz)_{b}-L₂-M Chemical Formula 4

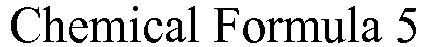

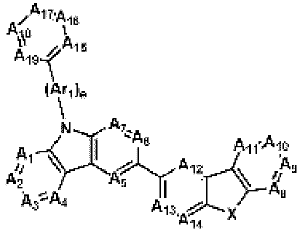

### BACKGROUND OF THE INVENTION

Among display devices, an electroluminescent device (EL device) is a self-luminescent display device, and has advantages of wider viewing angel as compared with LCD, excellent contrast, and fast response speed. An organic EL device, which uses aromatic diamine having a low molecular weight, and an aluminum complex, as a material for forming an electroluminescent layer, was first developed by Eastman Kodak Company in 1987 [Appl. Phys. Lett. 51, 913, 1987].

In the organic EL device, when charges are injected to an organic film formed between an electron injection electrode (cathode) and a hole injection electrode (anode), electrons and holes pair up and become extinct while light is emitted. The organic EL device has advantages in that elements can be formed on a flexible transparent substrate such as plastics, and it can be driven at a low voltage (10V or lower) as compared with a plasma display panel or an inorganic EL display. In addition, the organic EL device requires relatively small power consumption and has excellent color. Meanwhile, the organic EL device exhibits three colors, red, green, and blue, and thus it become an object of attention from many people as a next colorful display device.

An organic material for the organic EL device may be largely divided into an electroluminescent material and a charge transport material. The electroluminescent material is directly related to electroluminescent color and luminous efficiency, and requires several characteristics such as a high fluorescence quantum yield in a solid state, high mobility of electrons and holes, low degradability at the time of vacuum deposition, and uniform thin-film formability.

The electroluminescent material may be divided into a host material and a dopant material in view of function. In general, a device having a structure where an electroluminescent layer is formed by doping a host with dopants has been known to have excellent EL characteristic. Recently, development of organic EL device having high efficiency and long life property is becoming the most urgent issue, and particularly, development of materials more excellent than the existing electroluminescent materials is urgent, considering EL characteristic levels required for medium-large sized OLED panels. For this reason, the host material functioning as a solvent of solid state and an energy transferring member needs to have high purity, and appropriate molecular weight for enabling vacuum deposition. In addition, the host material needs to secure high thermal stability due to high glass transition temperature and high thermal decomposition temperature, and high electrochemical stability for long life property. Furthermore, the host material needs to be easy in the formation of amorphous thin film, and have excellent adhesive strength with materials of adjacent other layers while motion between layers need not occur.

A fluorescent material has been widely used until now, as the electroluminescent material functioning as the most important factor determining the luminous efficiency of the OLED, but development of a phosphorescent material is known to the best method that can improve the luminous efficiency theoretically up to four times in an electroluminescent mechanism.

As iridium (III) complex-based phosphor electroluminescent material, (acac)Ir(btp)2, Ir(ppy)3, and Firpic for respective red, green, and blue has been known until now. In recent, many phosphorescent materials are being studied in Korea, Japan, and Europe, and thus, more improved phosphor materials are expected to be known.

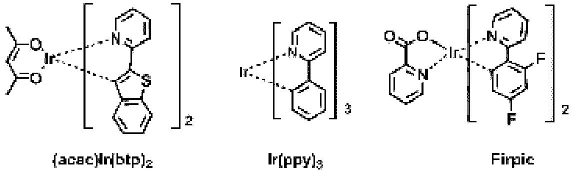

As a host material of a phosphorescence electroluminescent body, CBP is the most widely known until now, and a high-efficiency OLED to which a hole blocking layer of CBP or BAlq is applied is known.

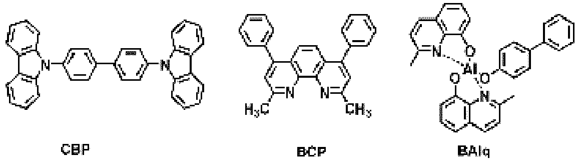

The existing materials are advantageous in view of electroluminescent characteristics. However, they may be deformed when subjected to a high-temperature depositing process under vacuum, due to a low glass transition temperature and inferior thermal stability thereof. Since power efficiency = (π/voltage) x current efficiency In OLED, power efficiency is inversely proportional to voltage. Therefore, power efficiency needs to be raised in order to lower power consumption of the OLED. An OLED using an actual phosphorescence electroluminescent material has a higher current efficiency (cd/A) as compared with an OLED using a fluorescence electroluminescent material. However, an OLED where the existing materials such as BAlq or CBP is used as a host of a phosphorescence electroluminescent material, has a higher driving voltage as compared with the OLED using a fluorescent material, and thus, large advantages are not present in view of power efficiency (lm/w).

### TECHNICAL PROBLEM

As the result of efforts made by the present inventors in order to solve the disadvantages of the prior art, an organic electroluminescent device having high color purity, high brightness, and long life property can be realized by interposing an organic layer, which includes an electroluminescent layer made by a combination of particular compounds, between an anode and a cathode on a substrate.

An object of the present invention is to provide an organic electroluminescent device where an organic layer is interposed between an anode and a cathode on a substrate, the organic layer including an electroluminescent layer containing one or more host compounds and one or more dopant compounds, and thus, an organic electroluminescent device having excellent luminous efficiency, high color purity, low driving voltage, and long life property.

### TECHNICAL SOLUTION

The present invention is directed to an organic electroluminescent device, and more specifically, an organic electroluminescent device where an organic layer is interposed between an anode and a cathode on a substrate, the organic layer including an electroluminescent layer containing one or more dopant compounds represented by Chemical Formula 1 below and one or more host compounds represented by Chemical Formulas 2 to 5 below. 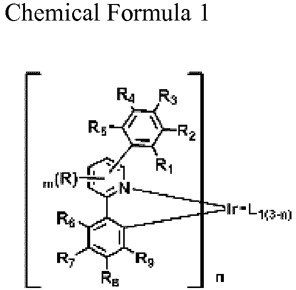 [wherein
L₁ represents an organic ligand;
R represents hydrogen, substituted or unsubstituted (C1-C30)alkyl, substituted or unsubstituted (C1-C30)alkoxy, substituted or unsubstituted (C6-C30)aryl or substituted or unsubstituted (C3-C30)heteroaryl;
R₁ through R₅ independently represent hydrogen, deuterium, halogen, substituted or unsubstituted (C3-C30)cycloalkyl, substituted or unsubstituted 5- to 7-membered heterocycloalkyl, cyano, nitro, BR₁₁R₁₂, PR₁₃R₁₄, P(=O)R₁₅R₁₆, R₁₇R₁₈R₁₉Si-, or substituted or unsubstituted (C6-C30)ar(C1-C30)alkyl;
R₆ through R₉ represent hydrogen, deuterium, halogen, substituted or unsubstituted (C1-C30)alkyl, substituted or unsubstituted (C1-C30)aryl, substituted or unsubstituted (C5-C30)heteroaryl, substituted or unsubstituted (C3-C30)cycloalkyl, substituted or unsubstituted 5- to 7-membered heterocycloalkyl, cyano, nitro, BR₁₁R₁₂, PR₁₃R₁₄, P(=O)R₁₅R₁₆, R₁₇R₁₈R₁₉Si-,NR₂₀R₂₁, R₂₂Y-, substituted or unsubstituted (C2-C30)alkenyl, substituted or unsubstituted (C2-C30)alkynyl, substituted or unsubstituted (C6-C30)ar(C1-C30)alkyl or they are linked to adjacent substituents to form a fused ring;
R₁₁ through R₂₂ independently represent substituted or unsubstituted (C1-C30)alkyl, substituted or unsubstituted (C6-C30)aryl or substituted or unsubstituted (C3-C30)heteroaryl;
Y represents S or O;
n and m independently represent an integer of 1 to 3;
the heterocycloalkyl and heteroaryl include one or more hetero atoms selected from the group consisting of B, N, O, S, P(=O), Si and P.] 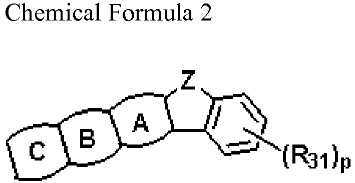 [wherein
Z represents -O-, -S-, -C(R₄₁R₄₂)-, -Si(R₄₃R₄₄)- or -N(R₄₅)-;
a ring A and a ring C independently represent 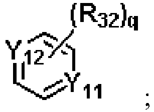 a ring B represents a ring of 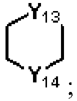 Y₁₁ through Y₁₂ independently represent C and N;
Y₁₃ through Y₁₄ independently represent a chemical bond, -O-, -S-, -C(R₄₁R₄₂)-, - Si(R₄₃R₄₄)- or -N(R₄₅)-; only except for the case where Y₁₃ and Y₁₄ represent a chemical bond at the same time;
R₃₁ and R₃₂ independently represent hydrogen, deuterium, halogen, substituted or unsubstituted (C1-C30)alkyl, substituted or unsubstituted (C6-C30)aryl, substituted or unsubstituted (C3-C30)heteroaryl, substituted or unsubstituted (C3-C30)cycloalkyl, substituted or unsubstituted 5- to 7-membered heterocycloalkyl, substituted or unsubstituted (C6-C30)ar(C1-C30)alkyl, substituted or unsubstituted (C1-C30)alkylsilyl group, substituted or unsubstituted (C1-C30)arylsilyl group, substituted or unsubstituted (C1-C30)alkyl(C6-C30)arylsilyl group, cyano, nitro, or hydroxyl, or they are linked to an adjacent substituent via substituted or unsubstituted (C3-C30)alkylene or (C3-C30)alkenylene with or without a fused ring to form an alicyclic ring and a monocyclic or polycyclic aromatic ring;
the R₄₁ through R₄₅ independently represent hydrogen, deuterium, halogen, substituted or unsubstituted (C1-C30)alkyl, substituted or unsubstituted (C6-C30)aryl, substituted or unsubstituted (C3-C30)heteroaryl, substituted or unsubstituted 5- to 7-membered heterocycloalkyl, substituted or unsubstituted (C3-C30)cycloalkyl or they are linked to adjacent substituents to form a ring;
p and q independently represent an integer of 0 to 4;
when p or q represent an integer larger than 2, each R₃₁ and R₃₂ may be the same or different from each other, and they may be linked to adjacent substituents to form a ring; and
the heterocycloalkyl and heteroaryl include one or more hetero atoms selected from the group consisting of B, N, O, S, P(=O), Si and P.]

Chemical Formula 3 **(Cz-L₂)ₐ-M**

Chemical Formula 4 **(Cz)_{b}-L₂-M**

[wherein
Cz is selected from following structures, 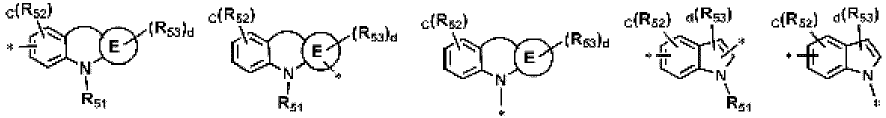 a ring E represents a (C6-C30)cycloalkyl group, a C6-C30)aryl group, or a (C5-C30)heteroaryl group;
R₅₁ through R₅₃ independently represent hydrogen, deuterium, halogen, substituted or unsubstituted (C1-C30)alkyl, substituted or unsubstituted (C6-C30)aryl, substituted or unsubstituted (C3-C30)heteroaryl, substituted or unsubstituted 5- to 7-membered heterocycloalkyl, substituted or unsubstituted (C6-C30)aryl fused with one or more substituted or unsubstituted (C3-C30)cycloalkyl, 5- to 7-membered heterocycloalkyl fused with one or more substituted or unsubstituted aromatic rings, substituted or unsubstituted (C3-C30)cycloalkyl, (C3-C30)cycloalkyl fused with one or more substituted or unsubstituted aromatic rings, substituted or unsubstituted (C6-C30)ar(C1-C30)alkyl, cyano, nitro, hydroxyl, BR₁₁R₁₂, PR₁₃R₁₄, P(=O)R₁₅R₁₆, R₁₇R₁₈R₁₉Si-, NR₂₀R₂₁, -YR₂₂ or they are linked to an adjacent substituent via substituted or unsubstituted (C3-C30)alkylene or substituted or unsubstituted (C3-C30)alkenylene with or without a fused ring to form an alicyclic ring and a monocyclic or polycyclic aromatic ring, carbon atoms of the formed alicyclic ring and monocyclic or polycyclic aromatic ring may be substituted with one or more hetero atoms selected from the group consisting of nitrogen, oxygen and sulfur; and
each R₅₂ or R₅₃ may be the same or different from each other;
L₂ represents a chemical bond, a substituted or unsubstituted (C6-C30)aryl group, or a substituted or unsubstituted (C5-C30)heteroaryl group;
M represents a substituted or unsubstituted (C6-C30)aryl group, or substituted or unsubstituted (C5-C30)heteroaryl;
a through d independently represent an integer of 0 to 4.] 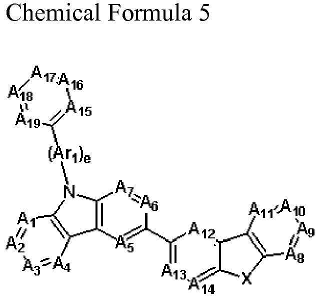 [wherein
A₁ through A₁₉ independently represent CR₆₁ or N;
X represents -C(R₆₂R₆₃)-, -N(R₆₄)-, -S-, -O-, -Si(R₆₅)(R₆₆)-, -P(R₆₇)-, -P(=O)(R₆₈)- or - B(R₆₉)-;
Ar₁ represents substituted or unsubstituted (C6-C40)arylene, or substituted or unsubstituted (C3-C40)heteroarylene; only except for the case where e=0 and A₁₅ through A₁₉ are CR₆₁ at the same time,
R₆₁ through R₆₉ independently represent hydrogen, deuterium, halogen, substituted or unsubstituted (C1-C30)alkyl, substituted or unsubstituted (C6-C30)aryl, substituted or unsubstituted (C6-C30)aryl fused with one or more substituted or unsubstituted (C3-C30)cycloalkyl, substituted or unsubstituted (C3-C30)heteroaryl, substituted or unsubstituted 5- to 7-membered heterocycloalkyl, 5- to 7-membered heterocycloalkyl fused with one or more substituted or unsubstituted aromatic rings, substituted or unsubstituted (C3-C30)cycloalkyl, substituted or unsubstituted fused with one or more aromatic rings (C3-C30)cycloalkyl, cyano, trifluoromethyl, NR₇₁R₇₂, BR₇₃R₇₄, PR₇₅R₇₆, P(=O)R₇₇R₇₈, R₇₉R₈₀R₈₁Si-, R₈₂Y₂₁-, R₈₃C(=O)-, R₈₄C(=O)O-, substituted or unsubstituted (C6-C30)ar(C1-C30)alkyl, substituted or unsubstituted (C2-C30)alkenyl, substituted or unsubstituted (C2-C30)alkynyl, carboxyl, nitro, or hydroxyl, or they are linked to an adjacent substituent via substituted or unsubstituted (C3-C30)alkylene or substituted or unsubstituted (C3-C30)alkenylene with or without a fused ring to form an alicyclic ring, a monocyclic or polycyclic aromatic ring, or a hetero aromatic ring;
the heterocycloalkyl and heteroaryl include one or more hetero atoms selected from the group consisting of B, N, O, S, P(=O), Si and P;
the R₇₁ through R₇₈ independently represent substituted or unsubstituted (C1-C30)alkyl, substituted or unsubstituted (C6-C30)aryl or substituted or unsubstituted (C3-C30)heteroaryl,
the R₇₉ through R₈₁ independently represent substituted or unsubstituted (C1-C30)alkyl or substituted or unsubstituted (C6-C30)aryl,
the Y₂₁ represents S or O,
R₈₂ represents substituted or unsubstituted (C1-C30)alkyl or substituted or unsubstituted (C6-C30)aryl,
the R₈₃ represents substituted or unsubstituted (C1-C30)alkyl, substituted or unsubstituted (C1-C30)alkoxy, substituted or unsubstituted (C6-C30)aryl or substituted or unsubstituted (C6-C30)aryloxy,
the R₈₄ represents substituted or unsubstituted (C1-C30)alkyl, substituted or unsubstituted (C1-C30)alkoxy, substituted or unsubstituted (C6-C30)aryl or substituted or unsubstituted (C6-C30)aryloxy, and
e represents an integer of 0 or 2.]

The organic electroluminescent device according to the present invention exhibits a host-dopant energy transfer mechanism, and thus, can express a certain high-efficiency electroluminescent performance, based on improved electron density distribution. Further, the organic electroluminescent device according to the present invention can overcome low initial efficiency, short operation life property, or the like, and secure high-performance electroluminescent performance with high efficiency and long life property for each color.

In the organic electroluminescent device of the present invention, the compound represented by Chemical Formula 1 included as a dopant may include the compounds represented by Chemical Formulas 6 and 7, the compound represented by Chemical Formula 2 included as a host may include the compounds represented by Chemical Formulas 8 to 13, and Cz of the Chemical Formulas 3 to 4 above may include the following structures, but are not limited thereto. 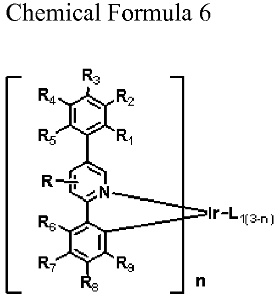 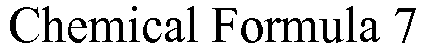 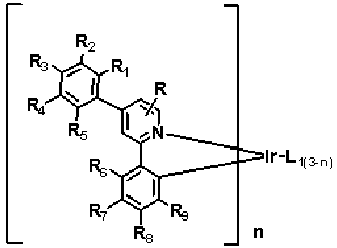 [wherein
R, R₁ through R₉, L₁ and n are the same as defined in Chemical Formula 1.] 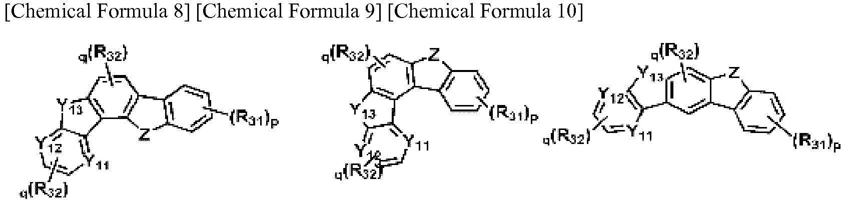 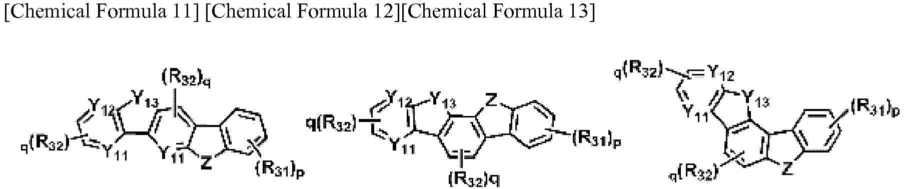 [wherein
R₃₁, R₃₂, Y₁₁ through Y₁₃, Z, p and q are the same as defined in Chemical Formula 2, wherein Cz of Chemical Formulas 3 to 4 is selected from following structures, and 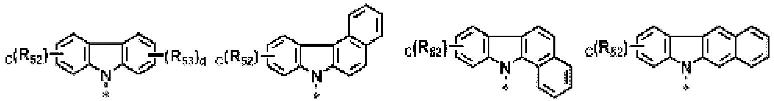 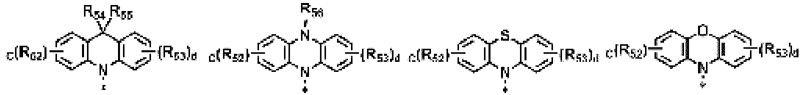 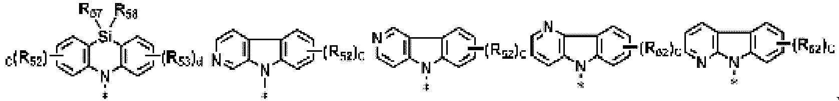 wherein
R₅₂, R₅₃, c and d are the same as defined in Chemical Formulas 3 to 4; and
R₅₄ through R₅₈ independently represent halogen, substituted or unsubstituted (C1-C30)alkyl, substituted or unsubstituted (C6-C30)aryl, or substituted or unsubstituted (C3-C30)heteroaryl or carbazolyl.]

Furthermore, L₁ represented by Chemical Formula 1 may be selected from following structures, but is not limited thereto. 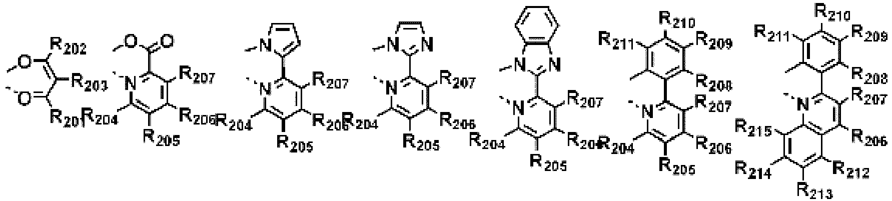 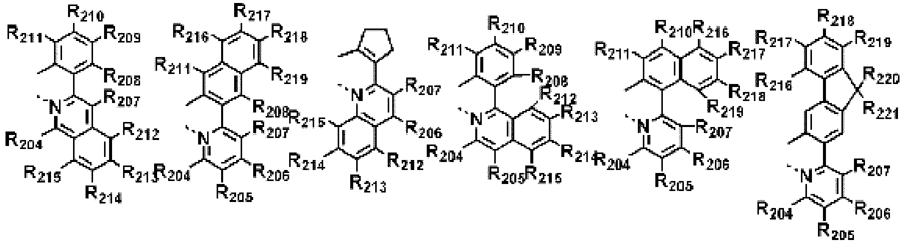 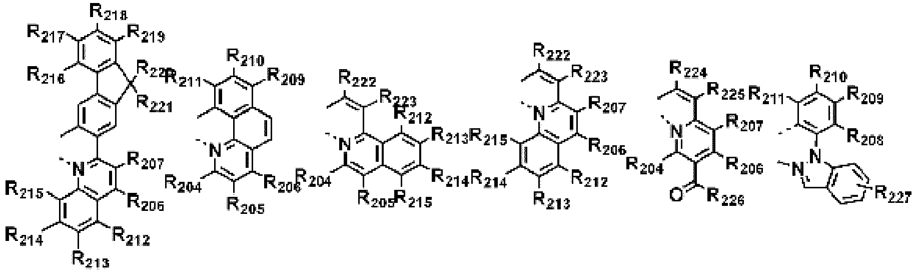 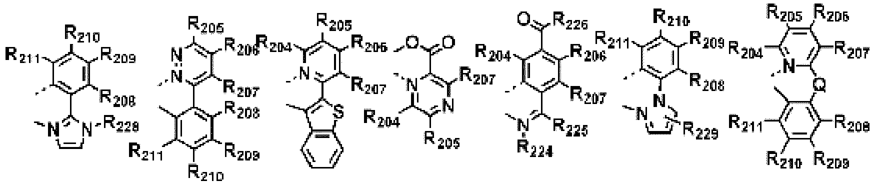 [wherein
the R₂₀, through R₂₀₃ independently represent hydrogen, deuterium, halogen-substituted or unsubstituted (C1-C30)alkyl, (C1-C30)alkyl-substituted or unsubstituted (C6-C30)aryl or halogen;
R₂₀₄ through R₂₁₉ independently represent hydrogen, deuterium, substituted or unsubstituted (C1-C30)alkyl, substituted or unsubstituted (C1-C30)alkoxy, substituted or unsubstituted (C3-C30)cycloalkyl, substituted or unsubstituted (C2-C30)alkenyl, substituted or unsubstituted (C6-C30)aryl, substituted or unsubstituted mono- or substituted or unsubstituted di-(C1-C30)alkylamino, substituted or unsubstituted mono or di-(C6-C30)arylamino, SF₅, substituted or unsubstituted tri(C1-C30)alkylsilyl, substituted or unsubstituted di(C1-C30)alkyl(C6-C30)arylsilyl, substituted or unsubstituted tri(C6-C30)arylsilyl, cyano or halogen;
R₂₂₀ through R₂₂₃ independently represent hydrogen, deuterium, halogen-substituted or unsubstituted (C1-C30)alkyl or (C1-C30)alkyl-substituted or unsubstituted (C6-C30)aryl;
R₂₂₄ and R₂₂₅ independently represent hydrogen, deuterium, substituted or unsubstituted (C1-C30)alkyl, substituted or unsubstituted (C6-C30)aryl or halogen, or R₂₂₄ and R₂₂₅ are linked via (C3-C12)alkylene or (C3-C12)alkenylene with or without a fused ring to form an alicyclic ring and a monocyclic or polycyclic aromatic ring;
R₂₂₆ represents substituted or unsubstituted (C1-C30)alkyl, substituted or unsubstituted (C6-C30)aryl, substituted or unsubstituted (C5-C30)heteroaryl or halogen;
R₂₂₇ through R₂₂₉ independently represent hydrogen, deuterium, substituted or unsubstituted (C1-C30)alkyl, substituted or unsubstituted (C6-C30)aryl or halogen;
Q represents 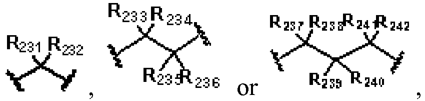 and
R₂₃₁ through R₂₄₂ independently represent hydrogen, deuterium, halogen-substituted or unsubstituted (C1-C30)alkyl, (C1-C30)alkoxy, halogen, substituted or unsubstituted (C6-C30)aryl, cyano, substituted or unsubstituted (C5-C30)cycloalkyl, or they are linked to an adjacent substituent via alkylene or alkenylene to form a spiro ring or a fused ring, or linked to R₂₀₇ or R₂₀₈ via alkylene or alkenylene to form a saturated or unsaturated fused ring.]

As described herein, the terms substitutions including "alkyl" "alkoxy", and, besides, "alkyl" moieties may include both linear and branched species, and "cycloalkyl" may include monocyclic hydrocarbon as well as polycyclic hydrocarbon such as substituted or unsubstituted adamantyl or substituted or unsubstituted (C7-C30)bicycloalkyl. As described herein, the term "aryl" means an organic radical derived from aromatic hydrocarbon by the removal of one hydrogen atom, and may include a single ring or a fused ring containing properly 4 to 7 ring atoms, preferably 5 or 6 ring atoms, and even may include a structure where a plurality of aryls are linked by single bonds. Specific examples thereof include phenyl, naphthyl, biphenyl, terphenyl, anthryl, indenyl, fluorenyl, phenanthryl, triphenylenyl, pyrenyl, perylenyl, chrysenyl, naphthacenyl, fluoranthenyl, or the like, but are not limited thereto. The naphthyl includes 1-naphthyl and 2-naphthyl, and the anthryl includes 1-anthryl, 2-anthryl and 9-anthryl. The phenanthryl includes 1-phenanthryl, 2-phenanthryl, 3-phenanthryl, 4-phenanthryl, and 9-phenanthryl, and the naphthacenyl includes 1-naphthacenyl, 2-naphthacenyl, and 9-naphthacenyl. The pyrenyl includes 1-pyrenyl, 2-pyrenyl, and 4-pyrenyl, and the biphenyl includes 2-biphenyl, 3-biphenyl, and 4-biphenyl. The terphenyl includes p-terphenyl-4-yl group, p-terphenyl-3-yl group, p-terphenyl-2-yl group, m-terphenyl-4-yl group, m-terphenyl-3-yl group, and m-terphenyl-2-yl group.

The fluorenyl includes 1-fluorenyl, 2-fluorenyl, 3-fluorenyl, 4-fluorenyl and 9-fluorenyl. The 'heteroaryl' described herein means an aryl group which contains 1 to 4 heteroatoms selected from B, N, O, S, P(=O), Si and P as aromatic ring backbone atoms and the remaining aromatic ring backbone atom is carbon. It may be 5- or 6-membered monocyclic heteroaryl or polycyclic heteroaryl condensed with one or more benzene rings, and may be partially saturated. In the present invention, "heteroaryl" include a form where one or more heteroaryls are liked by single bonds. The heteroaryl group includes a divalent aryl group wherein the heteroatom(s) in the ring may be oxidized or quaternized to form, for example, N- oxide or quaternary salt. Specific examples include monocyclic heteroaryl such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl, or the like, polycyclic heteroaryl such as benzofuranyl, benzothiophenyl, dibenzofuranyl, dibenzothiopenyl, isobenzofuranyl, benzimidazolyl, benzothiazolyl, benzoisothiazolyl, benzoisoxazolyl, benzoxazolyl, isoindolyl, indolyl, indazolyl, benzoth- iadiazolyl, quinolyl, isoquinolyl, cinnolinyl, quinazolinyl, quinoxalinyl, carbazolyl, phenanthridinyl, benzodioxolyl, acridinyl, phenanthrolinyl, phenazinyl, phenothiazinyl, phenoxazinyl or the like, N-oxide thereof (e.g., pyridyl N-oxide, quinolyl N-oxide), quaternary salt thereof, and the like, but are not limited thereto. The pyrrolyl includes 1-pyrrolyl, 2-pyrrolyl, and 3-pyrrolyl; the pyridyl includes 2-pyridyl, 3-pyridyl, and 4-pyridyl; the indolyl includes 1-indolyl, 2-indolyl, 3-indolyl, 4-indolyl, 5-indolyl, 6-indolyl, and 7-indolyl; the isoindolyl includes 1-isoindolyl, 2-isoindolyl, 3-isoindolyl, 4-isoindolyl, 5-isoindolyl, 6-isoindolyl, and 7-isoindolyl; the furyl includes 2-furyl, and 3-furyl; the benzofuranyl includes 2-benzofuranyl, 3-benzofuranyl, 4-benzofuranyl, 5-benzofuranyl, 6-benzofuranyl, and 7-benzofuranyl; the isobenzofuranyl includes 1-isobenzofuranyl, 3-isobenzofuranyl, 4-isobenzofuranyl, 5-isobenzofuranyl, 6-isobenzofuranyl, and 7-isobenzofuranyl; the quinolyl includes 3-quinolyl, 4-quinolyl, 5-quinolyl, 6-quinolyl, 7-quinolyl, and 8-quinolyl; the isoquinolyl includes 1-isoquinolyl, 3-isoquinolyl, 4-isoquinolyl, 5-isoquinolyl, 6-isoquinolyl, 7-isoquinolyl, and 8-isoquinolyl group; the qunoxalinyl includes 2-qunoxalinyl, 5-qunoxalinyl, and 6-qunoxalinyl; the carbazolyl includes 1-carbazolyl, 2-carbazolyl, 3-carbazolyl, 4-carbazolyl, and 9-carbazolyl; the phenanthrinyl includes 1-phenanthrinyl, 2-phenanthrinyl, 3-phenanthrinyl, 4-phenanthrinyl, 6-phenanthrinyl, 7-phenanthrinyl, 8-phenanthrinyl, 9-phenanthrinyl, and 10-phenanthrinyl; the acridinyl includes 1-acridinyl, 2-acridinyl, 3-acridinyl, 4-acridinyl, and 9-acridinyl; the phenanthrolinyl includes 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1, 8-phenanthrolin-6-yl group, 1, 8-phenanthrolin-7-yl group, 1, 8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, and 2,7-phenanthrolin-10-yl group; the phenazinyl includes 1-phenazinyl, and 2-phenazinyl; the phenothiazinyl includes 1-phenothiazinyl, 2-phenothiazinyl, 3-phenothiazinyl, 4-phenothiazinyl, and 10-phenothiazinyl; the phenoxazinyl includes 1-phenoxazinyl, 2-phenoxazinyl, 3-phenoxazinyl, 4-phenoxazinyl, and 10-phenoxazinyl; the oxazolyl includes 2-oxazolyl, 4-oxazolyl, and 5-oxazolyl; the oxadiazole includes 2-oxadiazolyl, and 5-oxadiazolyl; the furazanyl includes 3-furazanyl; the dibenzofuranyl includes 1-dibenzofuranyl, 2-dibenzofuranyl, 3-dibenzofuranyl, and 4-dibenzofuranyl; and the dibenzothiophenyl includes 1-dibenzothiophenyl, 2-dibenzothiophenyl, 3-dibenzothiophenyl, and 4-dibenzothiophenyl.As described herein, the term "(C1-C30)alkyl" may include (C1-C20)alkyl or (C1-C10)alkyl, and the term "(C6-C30)aryl" may include (C6-C20)aryl or (C6-C12)aryl. The term "(C3-C30)heteroaryl" may include (C3-C20)heteroaryl or (C3-C12)heteroaryl, and the term "(C3-C30)cycloalkyl" may include (C3-C20)cycloalkyl or (C3-C7)cycloalkyl. The term, "(C2-C30)alkenyl or alkynyl" may include (C2-C20)alkenyl or alkynyl, or (C2-C10)alkenyl or alkynyl.

As described herein, the expression "substituted" in "substituted or unsubstituted", means to be further substituted with an unsubstituted substituent. Herein, substituents further substituted with the R, R₁ through R₉, R₁₁ through R₁₂, R₃₁ through R₃₂, R₄₁ through R₄₅, R₅₁ through R₅₃, R₆₁ through R₆₉, R₇₁ through R₈₄, L₂₅ M and Ar₁ independently represent one or more selected from the group consisting of deuterium, halogen, halogen-substituted or unsubstituted (C1-C30)alkyl, (C6-C30)aryl, (C6-C30)aryl-substituted or unsubstituted (C3-C30)heteroaryl, 5- to 7-membered heterocycloalkyl, 5- to 7-membered heterocycloalkyl fused with one or more aromatic rings, (C3-C30)cycloalkyl, (C6-C30)cycloalkyl fused with one or more aromatic rings, R₉₁R₉₂R₉₃Si-, (C2-C30)alkenyl, (C2-C30)alkynyl, cyano, carbazolyl, NR₉₄R₉₅, BR₉₆R₉₇, PR₉₈R₉₉, P(=O)R₁₀₀R₁₀₁, (C6-C30)ar(C1-C30)alkyl, (C1-C30)alkyl(C6-C30)aryl, R₁₀₂S-, R₁₀₃O-, R₁₀₄C(=O)-, R₁₀₅C(=O)O-, carboxyl, nitro or hydroxyl; R₉₁ through R₁₀₃ independently represent hydrogen, deuterium, halogen, substituted or unsubstituted (C1-C30)alkyl, substituted or unsubstituted (C6-C30)aryl, substituted or unsubstituted (C3-C30)heteroaryl, or substituted or unsubstituted 5- to 7-membered heterocycloalkyl or they are linked to an adjacent substituent via substituted or unsubstituted (C3-C30)alkylene or substituted or unsubstituted (C3-C30)alkenylene with or without a fused ring to form an alicyclic ring and a monocyclic or polycyclic aromatic ring, carbon atoms of the formed alicyclic ring and monocyclic or polycyclic aromatic ring may be substituted with one or more hetero atoms selected from the group consisting of nitrogen, oxygen and sulfur; andR₁₀₄ and R₁₀₅ represent (C1-C30)alkyl, (C1-C30)alkoxy, (C6-C30)aryl or (C6-C30)aryloxy.

In particular, it is confirmed that when a compound of Chemical Formula 14 was selected as a dopant and compounds of Chemical Formula 8 and 10, Chemical Formula 11 to 12, and Chemical Formula 3 to 5 were selected as a host, more efficient luminous properties are revealed to obtain a high-performance organic electroluminescent device with high efficiency and long life property. 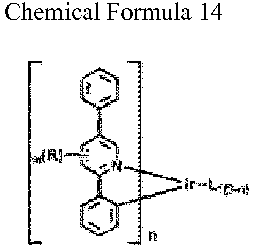 wherein
R represents substituted or unsubstituted (C1-C30)alkyl, substituted or unsubstituted (C6-C30)aryl;
L₁ is selected from the following structures, and 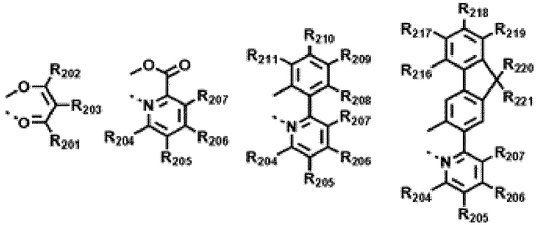 the R₂₀₁ through R₂₀₃ independently represent hydrogen, deuterium, halogen-substituted or unsubstituted (C1-C30)alkyl, (C1-C30)alkyl-substituted or unsubstituted (C6-C30)aryl or halogen;
R₂₀₄ through R₂₁₉ independently represent hydrogen, deuterium, substituted or unsubstituted (C1-C30)alkyl, substituted or unsubstituted (C6-C30)aryl, SF₅, substituted or unsubstituted tri(C1-C30)alkylsilyl, substituted or unsubstituted di(C1-C30)alkyl(C6-C30)arylsilyl, substituted or unsubstituted tri(C6-C30)arylsilyl, cyano or halogen;
R₂₂₀ through R₂₂₁ independently represent hydrogen, deuterium, halogen-substituted or unsubstituted (C1-C30)alkyl or (C1-C30)alkyl-substituted or unsubstituted (C6-C30)aryl, and
n, and m independently represent an integer of 1 to 3. 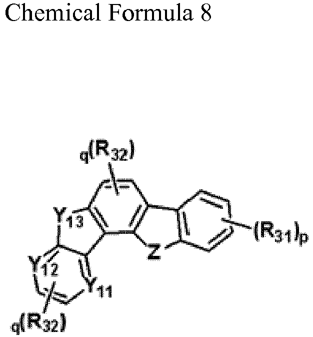 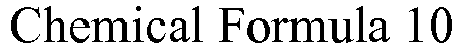 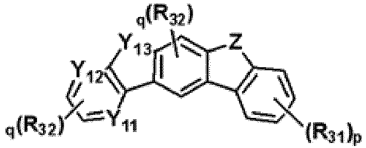 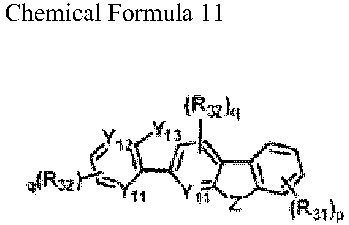 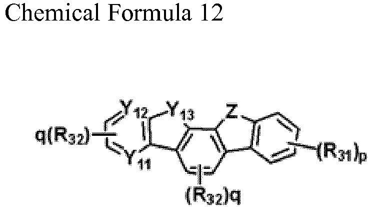 wherein
Z represents -O-, -S-, -C(R₄₁R₄₂)-, -N(R₄₅)-;
Y₁₁ through Y₁₂ represent C;
Y₁₃ represents -N(R₄₅)-;
R₃₁ and R₃₂ independently represent hydrogen, deuterium, halogen, substituted or unsubstituted (C1-C30)alkyl, substituted or unsubstituted (C6-C30)aryl, substituted or unsubstituted (C3-C30)heteroaryl, substituted (C1-C30)alkylsilyl group, substituted or unsubstituted (C1-C30)arylsilyl group, and substituted or unsubstituted (C1-C30)alkyl(C6-C30)arylsilyl group,
the R₄₁ through R₄₂ independently represent substituted or unsubstituted (C1-C30)alkyl, substituted or unsubstituted (C6-C30)aryl, and substituted or unsubstituted (C3-C30)heteroaryl,
the R₄₅ represents unsubstituted (C1-C30)alkyl, substituted or unsubstituted (C6-C30)aryl, and substituted or unsubstituted (C3-C30)heteroaryl,
p and q independently represent an integer of 0 through 4; when p or q represent an integer larger than 2, each R₃₁ and R₃₂ may be the same or different from each other.

Chemical Formula 3 (Cz-L₂)ₐ-M

Chemical Formula 4 (Cz)_{b}-L₂-M

wherein
Cz has a following structure, 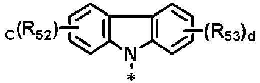 R₅₂ through R₅₃ independently represent hydrogen, deuterium, halogen, substituted or unsubstituted (C1-C30)alkyl, substituted or unsubstituted (C6-C30)aryl, substituted or unsubstituted (C3-C30)heteroaryl, R₁₇R₁₈R₁₉Si-, R₁₇ through R₁₉ independently represent substituted or unsubstituted (C1-C30)alkyl, substituted or unsubstituted (C6-C30)aryl; each R₅₂ or R₅₃ may be the same or different from each other;
L₂ represents a chemical bond, substituted or unsubstituted (C6-C30)arylene, and substituted or unsubstituted (C5-C30)heteroarylene;
M represents substituted or unsubstituted (C6-C30)aryl group, and substituted or unsubstituted (C5-C30)heteroaryl;
a through d independently represent an integer of 0 through 4. 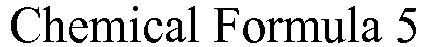 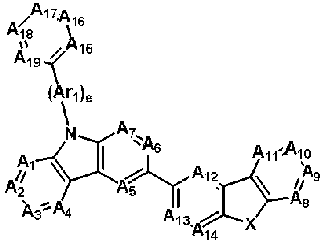 wherein
A₁ through A₁₄ represent CR₆₁;
A₁₅ through A19 independently represent CR₆₁ or N,
X represents -N(R₆₄)-, -S-, -O-, and -Si(R₆₅)(R₆₆)-;
Ar₁ represents substituted or unsubstituted (C6-C40)arylene, and substituted or unsubstituted (C3-C40)heteroarylene; except for the case that e=0 and A₁₅ through A₁₉ represent CR₆₁ at the same time,
R₆₁ and R₆₄ through R₆₆ independently represent hydrogen, deuterium, halogen, substituted or unsubstituted (C1-C30)alkyl, substituted or unsubstituted (C6-C30)aryl, substituted or unsubstituted (C3-C30)heteroaryl, NR₇₁R₇₂, and R₇₉R₈₀R₈₁Si-,
the R₇₁ through R₇₂ independently represent substituted or unsubstituted (C1-C30)alkyl, substituted or unsubstituted (C6-C30)aryl or substituted or unsubstituted (C3-C30)heteroaryl, and the R₇₉ through R₈₁ independently represent substituted or unsubstituted (C1-C30)alkyl or substituted or unsubstituted (C6-C30)aryl, and e represents an integer of 0 through 2.The organic electroluminescent compound of Chemical Formula 1 may be exemplified by the following compounds, which are not intended to limit the present invention.

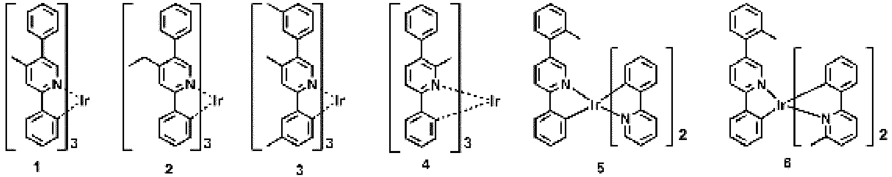

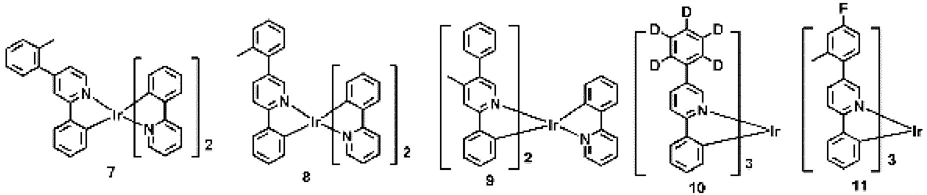

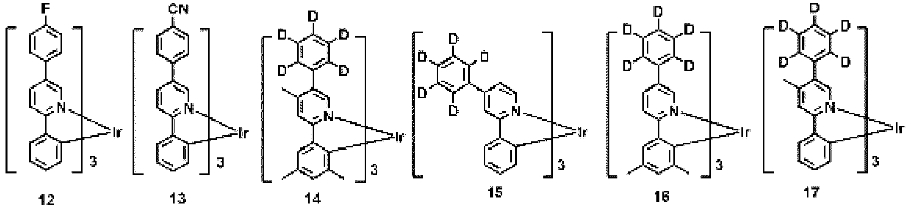

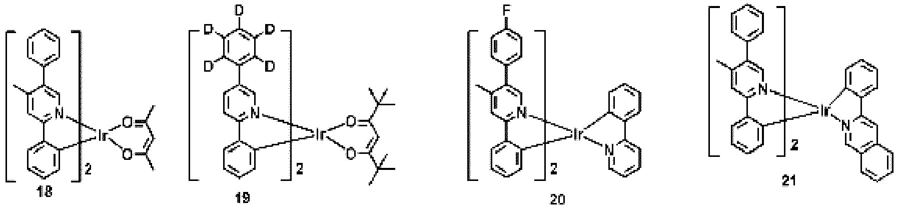

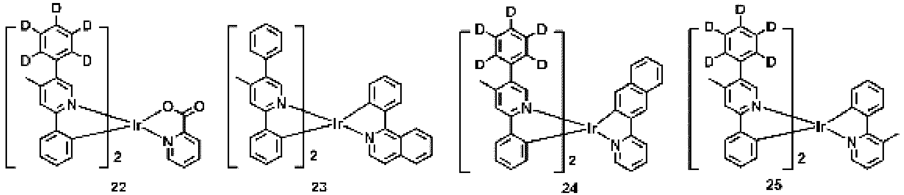

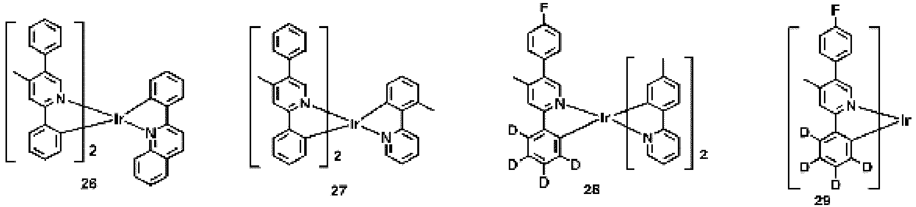

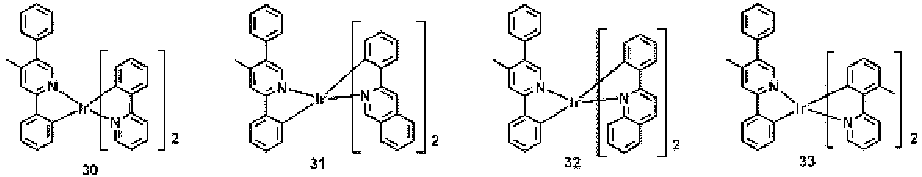

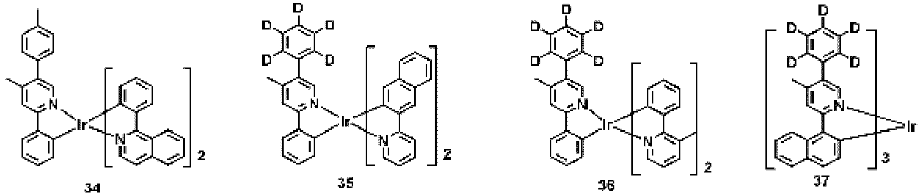

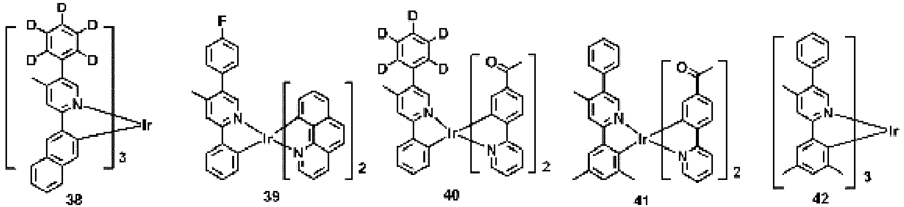

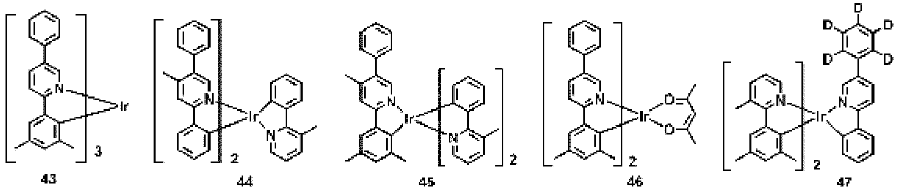

The organic electroluminescent compounds of Chemical Formulas 2 to 5 may be exemplified by the following compounds, which are not intended to limit the present invention.

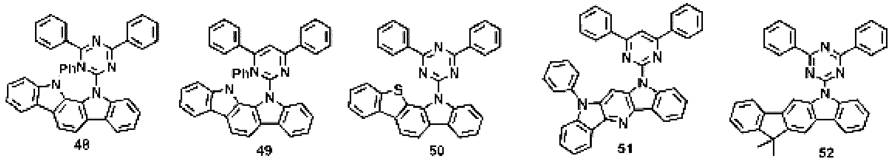

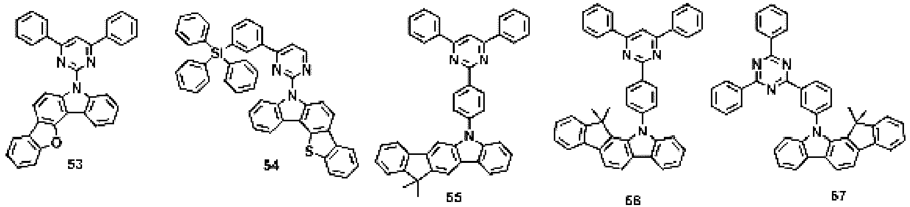

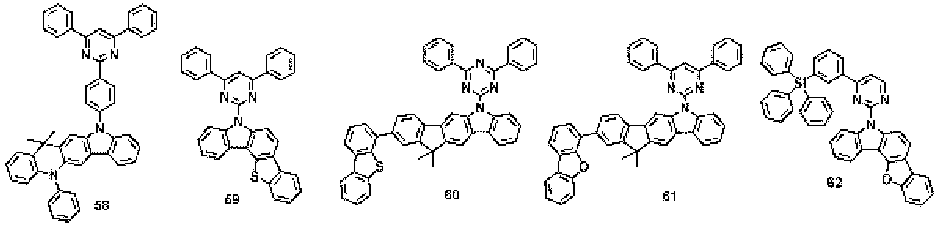

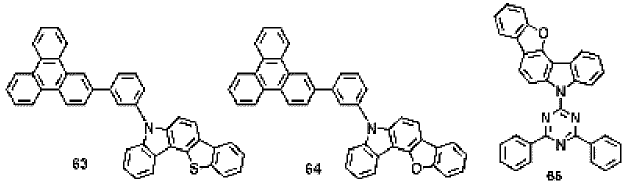

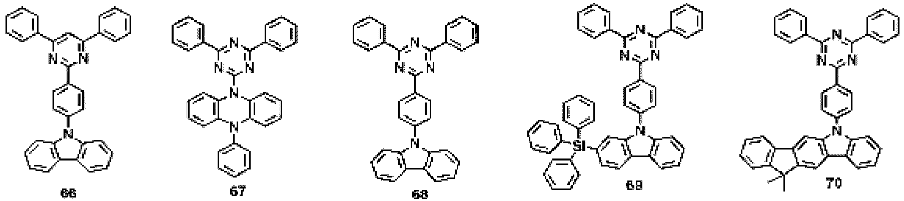

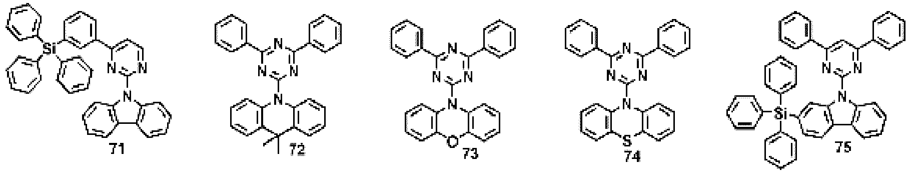

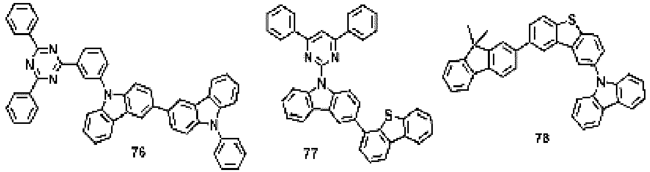

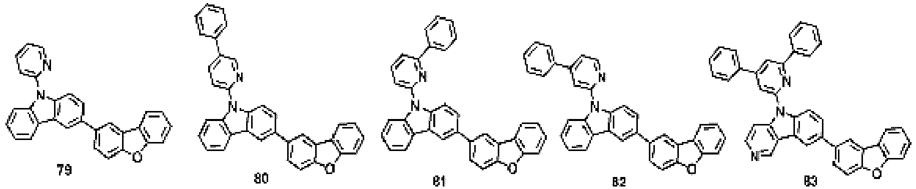

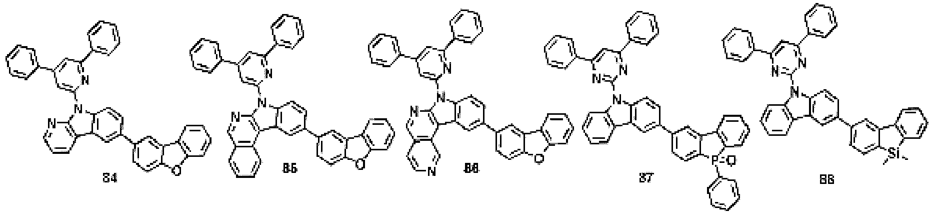

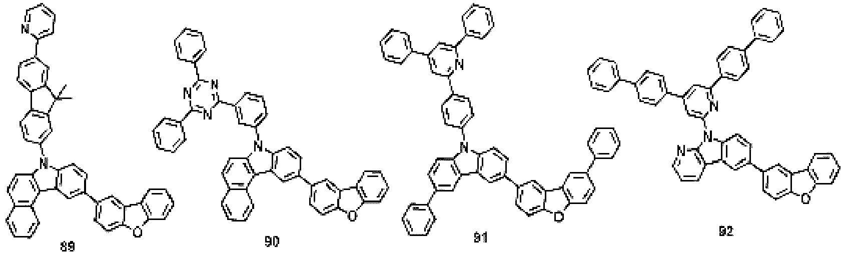

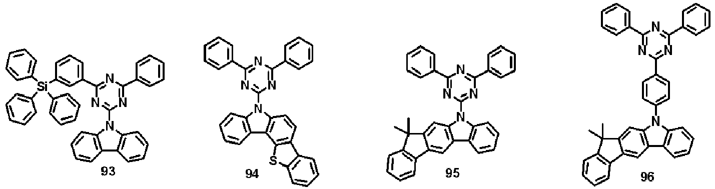

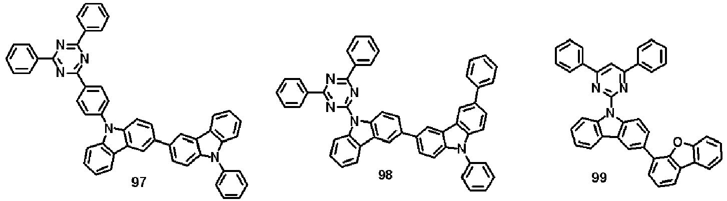

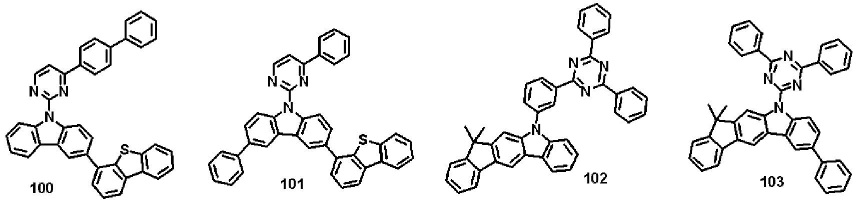

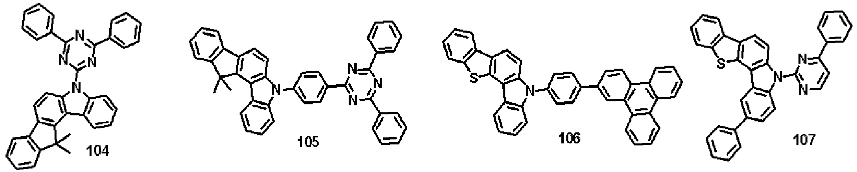

The compound of Chemical Formula 1 may be prepared as shown in Scheme 1. The following preparing method is not intended to limit a method for preparing the organic electroluminescent compound of Chemical Formula 1, and modifications of the following preparing method would be obvious to those skilled in the art.

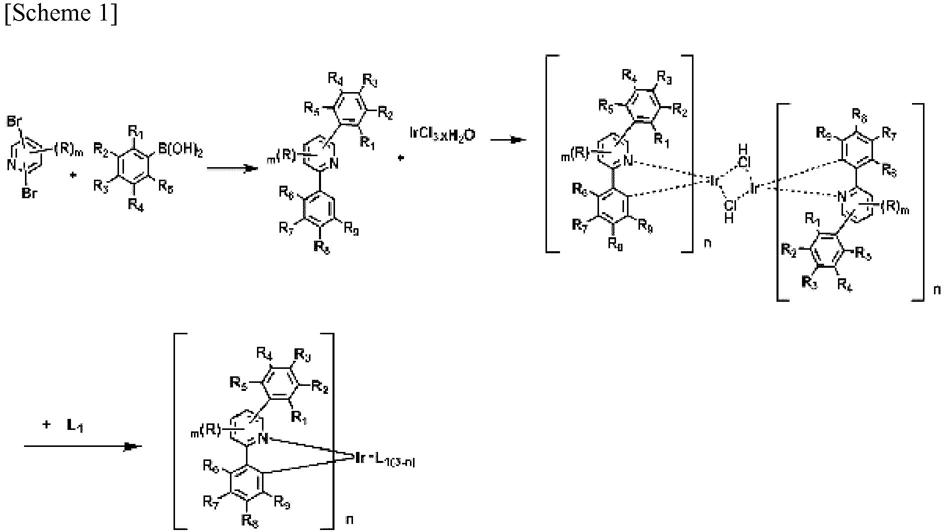

[In Scheme 1, L₁, R, R₁ through R₉, n and m are the same as defined in Chemical Formula 1.]

The electroluminescent layer means a layer where electroluminescence occurs. It may have a single layer or a plurality of layers where two or more layers are stacked. In case a combination of a dopant and a host is used in the present invention, remarkable improvement in luminous efficiency may be confirmed.

In addition, a doping concentration of the dopant compound based on the host compound in the electroluminescent layer is in a range of below 20 wt%.

In the organic electroluminescent device of the present invention, the organic layer may further contain one or more metals selected from the group consisting of organic metals of Group 1, Group 2, 4^{th} period and 5^{th} period transition metals, lanthanide metals, and d-transition elements in the Periodic Table of Elements or complex compounds, besides the organic electroluminescent compounds of Chemical Formulas 1 to 4. The organic layer may include both an electroluminescent layer and a charge generating layer.

In the organic electronic device of the present invention, the organic layer may include the organic electroluminescent compounds of Chemical Formulas 1 to 4, and at the same time include one or more compounds selected from the group consisting of arylamine-based compounds and styrylarylamine-based compounds. Specific examples of the arylamine-based compounds or the styrylarylamine-based compounds are described on paragraphs <212> to <224> in the specification of Korean Patent Application No. 10-2008-0060393, but are not limited thereto. Specific examples of the organic electroluminescent compounds of Chemical Formulas 2 to 4 are exemplified in Korean Patent Application Nos. 10-2009-0027221, 10-2009-0027256, 10-2009-0037519, 10-2009-0062882, 10-2009-0067370, 10-2009-0073260, 10-2009-0123174, 10-2010-0007866, or 10-2010-0040384, but are not limited thereto.

Further, the organic layer may further include one or more organic electroluminescent layers containing red, green, or blue electroluminescent compounds besides the organic electroluminescent compound at the same time, thereby manufacturing an organic electroluminescent device for emitting white light. The compounds for emitting red, green, or blue light are exemplified in Korean Patent Application Nos. 10-2008-0123276, 10-2008-0107606, or 10-2008-0118428, but are not limited thereto.

In the organic electroluminescent device of the present invention, it is preferable to arrange at least one layer (hereinafter, referred to as "surface layer") selected from chalcogenide layers, metal halide layers, and metal oxide layers, on the inside surface of at least one side of a pair of electrodes. Specifically, it is preferable to arrange a chalcogenide (including oxides) layer of silicon and aluminum metal on an anode surface of an electroluminescent medium layer, and a metal halide layer or a metal oxide layer on a cathode surface of the electroluminescent medium layer. This enables driving stability to be obtained. Examples of the chalcogenides may include SiO_{X}(1≤X≤2), AlO_{X}(1≤X≤1.5), SiON, SiAlON, and the like, examples of the metal halides may include LiF, MgF₂, CaF₂, rare earth metal fluoride, and the like, and examples of the metal oxides may include Cs₂O, Li₂O, MgO, SrO, BaO, CaO and the like.

In the organic electroluminescent device of the present invention, it is also preferable to arrange a mixed region of an electron transport compound and a reductive dopant or a mixed region of a hole transport compound and an oxidative dopant on a surface of at least one of the pair of electrodes thus manufactured. Therefore, the electron transport compound is reduced into an anion, which facilitates to inject or transport electrons into the electroluminescent medium from the mixed region. In addition, the hole transport compound is oxidized into a cation, which facilitates to inject or transport holes into the electroluminescent medium from the mixed region. Preferable oxidative dopants include various Lewis acids and acceptor compounds. Preferable reductive dopants include alkaline metals, alkaline metal compounds, alkaline earth metals, rare-earth metals, and mixtures thereof. In addition, a layer of the reductive dopant may be used as the charge generating layer to manufacture a white organic electroluminescent device having two or more electroluminescent layers.

### ADVANTAGEOUS EFFECTS

As set forth above, an organic electroluminescent device using a specific dopant-host organic electroluminescent compound according to the present invention had better luminous efficiency and longer operation life at a lower driving voltage as compared with a device using the existing electroluminescent material. It is considered that a specific combination of dopant and host has a comparative proper level of energy to show excellent luminous efficiency and long operation life.

### BEST MODE

Hereinafter, the present invention is further described by taking representative compounds of the present invention as examples with respect to the organic electroluminescent compound according to the invention, a preparing method thereof, and electroluminescent properties of the devices, but those examples are provided only for illustration of the embodiments, not being intended to limit the scope of the invention.

### [Preparation Example 1] Preparation of Compound 1

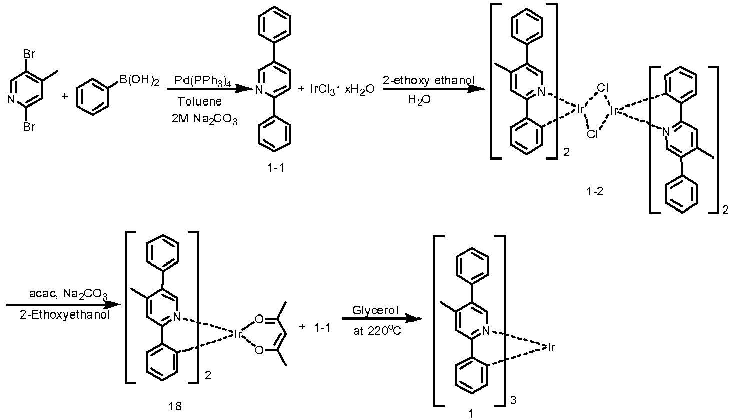

### Preparation of Compound 1-1

2,5-Dibromo-4-methylpyridine 30 g (120 mmol), phenylboronic acid 44 g (359 mmol), and Pd(PPh₃)₄ 8.3 g (7.17 mmol) were dissolved in toluene 400 mL, ethanol 200 mL, and 2M Na₂CO₃ 300 mL in the presence of nitrogen, and then stirred under reflux at 120 . Upon completion of the reaction after 2 hours, the resultant material was washed with distilled water, and extracted with ethyl acetate. The organic layer was dried over MgSO₄ and the solvent was removed by a rotary evaporator, followed by purification using column chromatography, thereby obtaining Compound 1-1 (15.3 g, 61 mmol).

### Preparation of Compound 1-2

Compound 1-2 20.1 g (82 mmol), iridium chloride (IrCl₃) 11 g (37 mmol), 2-ethoxyethanol 450 mL, and distilled water 150 mL were put into a reaction vessel, and then stirred under reflux for 24 hours. Upon completion of the reaction, the resultant material was cooled to room temperature, and the precipitated solid material was filtered. The solid thus obtained was washed with water and methanol, and recrystallized with hexane, thereby obtaining Compound **1-2** 17 g(12 mmol).

### Preparation of Compound 18

Compound **1-2** 17 g (12 mmol), Na₂CO₃ 7.6 g (71 mmol), and 2,4-pentanedione 3.7 ml (37 mmol) were dissolved in 2-ethoxy ethanol 200 mL, and then stirred under reflux for 5 hours. Upon completion of the reaction, the resultant material was cooled to room temperature, and the precipitated solid was filtered. The solid thus obtained was separated and recrystallized by using silica gel column chromatography, thereby obtaining yellow crystalline Compound **18** 13 g (17 mmol).

### Preparation of Compound 1

Compound **18** 8 g(10.2 mmol), Compound **1-1** 5 g(20 mmol), and glycerol 100 mL were stirred under reflux at 220°C for 12 hours, and then cooled to room temperature. The resultant mixture was washed with water and methanol, and then dissolved in methylene chloride, followed by separation using silica gel column chromatography, thereby obtaining yellow crystals of an iridium complex compound, Compound 1 6 g(7 mmol).
MS/FAB found 926, calculated 925.15

### [Preparation Example 2] Preparation of Compound 8

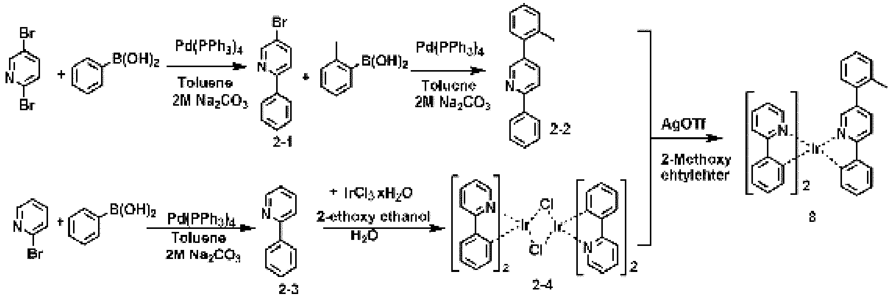

### Preparation of Compound 2-1

2,5-dibromopyridine 5 g (21 mmol) was treated in the same manner as the preparation method of Preparation Example 1 for Compound 1-1, thereby obtaining Compound 2-1 4 g (17 mmol).

### Preparation of Compound 2-2

Compound **2-1** 4 g (17 mmol), o-tolylboronic acid 2.8 g (20 mmol), and Pd(PPh₃)₄ 1g (0.9 mmol) were dissolved in toluene 80 mL, ethanol 40 mL, and 2M Na₂CO₃ 40 mL in the presence of nitrogen, and then stirred under reflux at 120 . Upon completion of the reaction after 2 hours, the resultant material was washed with distilled water, and extracted with ethyl acetate. The organic layer was dried over MgSO₄ and the solvent was removed by a rotary evaporator, followed by purification using column chromatography, thereby obtaining Compound **2-2** (4 g, 15 mmol).

### Preparation of Compound 2-3

2-Bromopyridine 5 g (21 mmol) was treated in the same manner as the preparation method of Preparation Example 1 for Compound 1-1, thereby obtaining Compound 2-3 4 g (17 mmol).

### Preparation of Compound 2-4

Compound 2-3 4 g(15 mmol), iridium chloride(IrCl₃) 2 g(6.8 mmol), 2-ethoxyethanol 90 mL, and distilled water 30 mL were added and stirred under reflux for 24 hours. Upon completion of the reaction, the resultant material was cooled to room temperature, and the precipitated solid was filtered. The solid thus obtained was washed with water and methanol, and recrystallized with hexane, thereby obtaining Compound **2-4** 3 g (2 mmol).

### Preparation of Compound 8

Compound **2-4** 14 g (13 mmol), Compound **2-2** 9.8 g(39 mmol), AgCF₃SO₃ 10 g (39 mmol), and 2-methoxy-ethylether 50 mL were stirred under reflex for 12 hours, and then cooled to room temperature. The resultant mixture was washed with water and methanol, and then dissolved in methanol, followed by separation using silica gel chromatography, thereby obtaining red crystals of an iridium complex compound, Compound **8** 3.5 g (4.5 mmol).
MS/FAB found 745, calculated 744.90

### [Preparation Example 3] Preparation of Compound 10

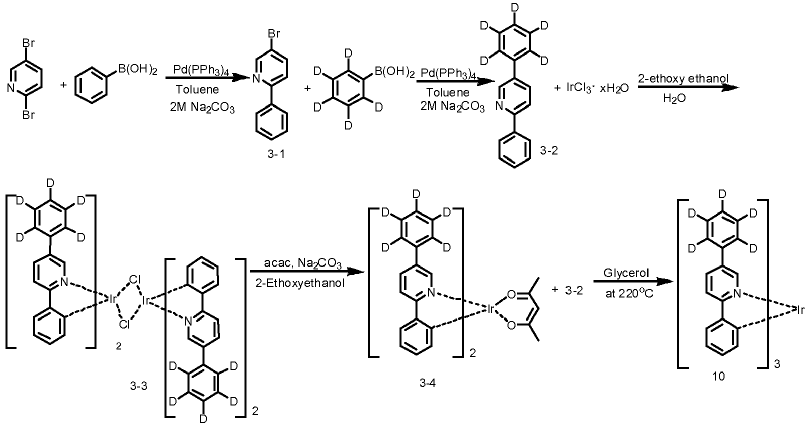

### Preparation of Compound 3-1

2.5-Dibromopyridine 5 g (21 mmol) was treated in the same manner as the preparation method of Preparation Example 1 for Compound 1-1, thereby obtaining Compound **3-1** 2.6 g (11 mmol).

### Preparation of Compound 3-2

Compound **3-1** 2.6 g (11 mmol), d⁵-phenyl boronic acid 1.8 g (13 mmol), and Pd(PPh₃)₄ 0.7 g (0.6 mmol) were dissolved in toluene 40 mL, ethanol 20 mL, and 2M Na₂CO₃ 20 mL in the presence of nitrogen, and then stirred under reflux at 120 . Upon completion of the reaction after 2 hours, the resultant material was washed with distilled water, and extracted with ethyl acetate. The organic layer was dried over MgSO₄ and the solvent was removed by a rotary evaporator, followed by purification using column chromatography, thereby obtaining Compound **3-2** (2.2 g, 9.3 mmol).

### Preparation of Compound 3-3

Compound **3-2** 2.2 g(9.3 mmol), iridium chloride(IrCl₃) 1.1 g(4.2 mmol), 2-ethoxyethanol 45 mL, and distilled water 15 mL were added and stirred under reflux for 24 hours. Upon completion of the reaction, the resultant material was cooled to room temperature, and the precipitated solid was filtered. The solid thus obtained was washed with water and methanol, and recrystallized with hexane, thereby obtaining Compound 3-3 1.9 g (1.4 mmol).

### Preparation of Compound 3-4

Compound **3-3** 1.9 g (1.4 mmol), Na₂CO₃ 0.82 g(8.4 mmol), and 2,4-pentanedione 2.3 ml(4.2 mmol) were dissolved in 2-ethoxyethanol 30 mL and then stirred under reflux for 5 hours. Upon completion of the reaction, the resultant material was cooled to room temperature, and the precipitated solid was filtered. The solid thus obtained was separated by using silica gel column chromatography and recrystralized, thereby obtaining yellow crystals, Compound **3-4** 0.67 g (0.9 mmol).

### Preparation of Compound 10

Compound **3-4** 1.4 g (1.8 mmol), Compound 3-2 0.85 g (3.6 mmol) and glycerol 20 mL were stirred under reflux for 12 hours at 220°C and cooled to room temperature. The mixture was washed with water and methanol, dissolved in methylenechloride and separated by using silica gel column chromatography, thereby obtaining yellow crystals of an iridium complex compound, Compound **10** 0.8 g(0.9 mmol).
MS/FAB found 899, calculated 898.16

### [Preparation Example 4] Preparation of Compound 20

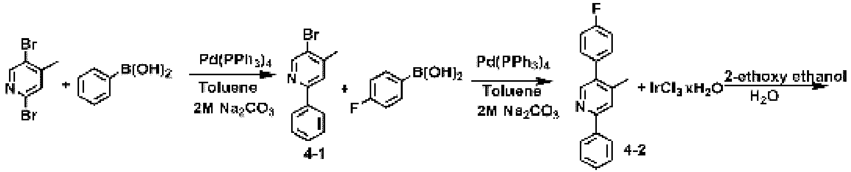

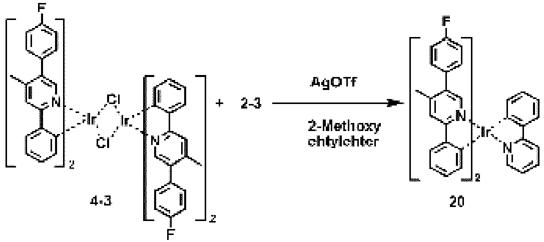

### Preparation of Compound 4-1

2,5-Dibromo-4-methylpyridine 5 g (21 mmol) was treated in the same manner as the preparation method of Preparation Example 1 for Compound 1-1, thereby obtaining Compound **4-1** 4 g (17 mmol).

### Preparation of Compound 4-2

Compound 4-1 4 g (17 mmol), 4-fluorophenylboronic acid 2.8 g (20 mmol), and Pd(PPh₃)4 1 g (0.9 mmol) were dissolved in toluene 80 mL, ethanol 40 mL, and 2M Na₂CO₃ 40 mL in the presence of nitrogen, and then stirred under reflux at 120 . Upon completion of the reaction after 2 hours, the resultant material was washed with distilled water, and extracted with ethyl acetate. The organic layer was dried over MgSO₄ and the solvent was removed by a rotary evaporator, followed by purification using column chromatography, thereby obtaining Compound **4-2** (4 g, 15 mmol).

### Preparation of Compound 4-3

Compound **4-2** 4 g(15 mmol), iridium chloride(IrCl₃) 2 g(6.8 mmol), 2-ethoxyethanol 90 mL, and distilled water 30 mL were added and stirred under reflux for 24 hours. Upon completion of the reaction, the resultant material was cooled to room temperature, and the precipitated solid was filtered. The solid thus obtained was washed with water and methanol, and recrystallized with hexane, thereby obtaining Compound **4-3** 3 g (2 mmol).

### Preparation of Compound 20

Compound 4-3 14 g (13 mmol), Compound **2-3** 9.8 g (39 mmol), AgCF₃SO₃ 10 g(39 mmol), 2-Methoxy-ethylether 50 mL were stirred under reflux for 12 hours and cooled to room temperature. The resultant material was washed with water and methanol, dissolved in methanol, and separated by using silica gel column chromatography, thereby obtaining red crystals of an iridium complex compound, Compound **20** 3.5 g(4.5 mmol).
MS/FAB found 782, calculated 871.01

### [Preparation Example 5] Preparation of Compound 37

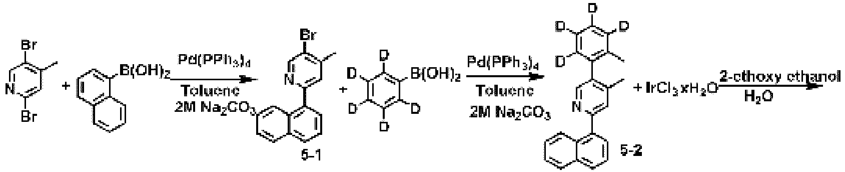

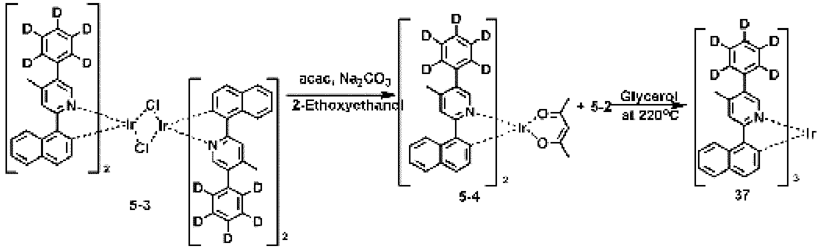

### Preparation of Compound 5-1

2,5-Dibromo-4-methylpyridine 5 g (21 mmol), naphthalene-1-ylboronic acid 2.6 g (21 mmol), and Pd(PPh₃)₄ 1.3 g (1.1 mmol) were dissolved in toluene 50 mL, ethanol 25 mL, and 2M Na₂CO₃ 25 mL in the presence of nitrogen, and then stirred under reflux at 120 . Upon completion of the reaction after 2 hours, the resultant material was washed with distilled water, and extracted with ethyl acetate. The organic layer was dried over MgSO₄ and the solvent was removed by a rotary evaporator, followed by purification using column chromatography, thereby obtaining Compound **5-1** (2.6 g, 11 mmol).

### Preparation of Compound 5-2

Compound **5-1** 2.6 g (11 mmol), d⁵-phenyl boronic acid 1.8 g (13 mmol), and Pd(PPh₃)₄ 0.7 g (0.6 mmol) were dissolved in toluene 40 mL, ethanol 20 mL, and 2M Na₂CO₃ 20 mL in the presence of nitrogen, and then stirred under reflux at 120 . Upon completion of the reaction after 2 hours, the resultant material was washed with distilled water, and extracted with ethyl acetate. The organic layer was dried over MgSO₄ and the solvent was removed by a rotary evaporator, followed by purification using column chromatography, thereby obtaining Compound 5-2 (2.2 g, 9.3 mmol).

### Preparation of Compound 5-3

Compound **5-2** 2.2 g(9.3 mmol), iridium chloride(IrCl₃) 1.1 g(4.2 mmol), 2-ethoxyethanol 45 mL, and distilled water 15 mL were added and stirred under reflux for 24 hours. Upon completion of the reaction, the resultant material was cooled to room temperature, and the precipitated solid was filtered. The solid thus obtained was washed with water and methanol, and recrystallized with hexane, thereby obtaining Compound 5-3 1.9 g (1.4 mmol).

### Preparation of Compound 5-4

Compound **5-3** 1.9 g (1.4 mmol), Na₂CO₃ 0.82 g(8.4 mmol), and 2,4-pentanedione 2.3 ml(4.2 mmol) were dissolved in 2-ethoxyethanol 30 mL and then stirred under reflux for 5 hours. Upon completion of the reaction, the resultant material was cooled to room temperature, and the precipitated solid was filtered. The solid thus obtained was separated by using silica gel column chromatography and recrystralized, thereby obtaining yellow crystals, Compound **5-4** 0.67 g (0.9 mmol).

### Preparation of Compound 37

Compound **5-4** 1.4 g (1.8 mmol), Compound **5-2,** and glycerol 20 mL were stirred under reflux for 12 hours at 220°C and cooled to room temperature. The mixture was washed with water and methanol, dissolved in methylenechloride and separated by using silica gel column chromatography, thereby obtaining yellow crystals of an iridium complex compound, Compound 37 0.8 g(0.9 mmol).
MS/FAB found 1091, calculated 1090.42

### [Preparation Example 6] Preparation of Compound 50

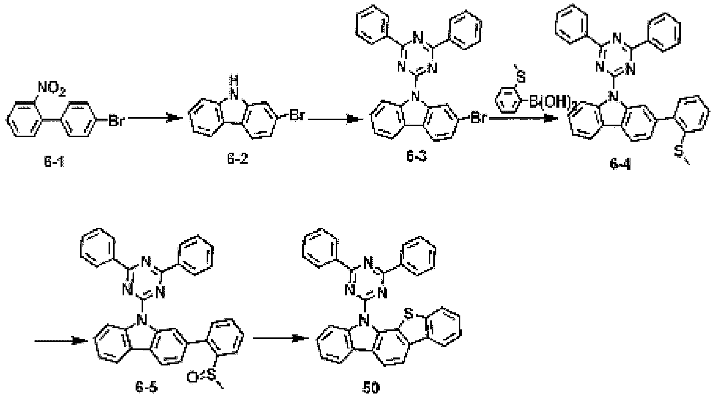

### Preparation of Compound 6-2

Compound **6-1** 28g (100.68mmol) was mixed with triethylphosphite 300ml, and then the mixture was stirred at 150 . After 6 hours, the resultant material was cooled to room temperature, and then distilled under reduced pressure, followed by extraction with EA and wash with distilled water. Then, drying over magnesium sulfate, distillation under reduced pressure, and then column separation were performed, thereby obtaining Compound 6-2 11g(44.69mmol, 44.38%).

### Preparation of Compound 6-3

Compound **6-2** 11g (44.69mmol), 2-chloro-4,6-diphenyl-1,3,5-triazine 18.23g (89.39mmol), CuI 4.25g(22.34mmol), K₃PO₄ 28.4g(134.09mmol), toluene 200ml were mixed, and then the mixture was heated to 50 . Ethylenediamine 3.01ml (44.69mmol) was put thereinto, and stirred under reflux. After 14 hours, the resultant material was cooled to room temperature, and then distilled water was added thereinto, followed by extraction with EA and drying over magnesium sulfate. Then the resultant material was distilled under reduced pressure, followed by column separation, thereby obtaining Compound **6-3** 12g(37.24mmol, 83.32%).

### Preparation of Compound 6-4

Compound **6-3** 12g (37.42mmol), 2-(methylthio)phenylboronic acid 7.5g (44.69mmol), Pd(PPh₃)₄ 2.15g (1.6mmol), 2M aqueous Na₂CO₃ solution 45ml, and THF 200ml were mixed, and then stirred under reflux. After 5 hours, the resultant material was cooled to room temperature, and then extracted with EA, followed by wash with distilled water. Then, drying over magnesium sulfate, distillation under reduced pressure, and then column separation were performed, thereby obtaining Compound 6-4 10g(27.36mmol, 73.47%).

### Preparation of Compound 6-5

Compound **6-4** 10g (27.36mmol) was added into acetic acid 100ml, and H₂O₂ 2.65ml (30.09mmol), 35%) was slowly added thereinto. The mixture was stirred at room temperature for 12 hours, and the acetic acid was distilled under reduced pressure. The resultant was extracted with dichloromethane, and neutralized with aqueous NaHCO₃ solution. Then, drying over magnesium sulfate and distillation under reduced pressure were performed, thereby obtaining Compound 6-5 10g(26.21mmol, 95.79%).

### Preparation of Compound 50

Compound **6-5** 10g (26.21mmol) was mixed with trifluoromethanesulfonic acid 70ml, and then the mixture was stirred to 100 . After 5 hours, the resultant material was cooled to room temperature, and then added into pyridine-distilled water (in the ratio of 1:5) 100 ml. The resultant material was stirred under reflux for 1 hour, and cooled to room temperature. The generated solid was filtered under reduced pressure, followed by column separation, thereby obtaining Compound **50** 6g(17.16mmol, 65.47%).
MS/FAB found 505, calculated 504.60

### [Preparation Example 7] Preparation of Compound 52

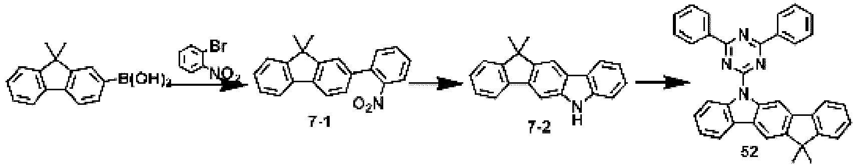

### Preparation of Compound 7-1

1-Bromo-2-nitrobenzene 15g (0.074mol) was added into a 1 L two-neck RBF, and 9,9-dimethyl-9H-fluoren-2-ylboronic acid 23g (0.096mol), Pd(PPh₃)₄ 4.2g (0.003mol), Na₂CO₃ (2M) 111mL, and EtOH 111mL were added thereinto, and toluene 200ml was added thereinto. Then, the resultant mixture was heated to 120 , and then stirred for 3 hours. Upon completion of the reaction, the resultant material was washed with distilled water, and extracted with ethyl acetate. The organic layer was dried over MgSO₄ and the solvent was removed by a rotary evaporator, followed by purification using column chromatography, thereby obtaining Compound 7-1 22g (95%).

### Preparation of Compound 7-2

Compound 7-1 24g (0.076mol) was put into a 1L two-neck RBF, and triethylphosphoite 200ml and 1,2-dichlorobenzene 200ml were added thereinto. The resultant mixture was heated to 140 , and then stirred for 12 hours. Upon completion of the reaction, the solvent was distilled, followed by wash with distilled water and extraction with ethyl acetate. The organic layer was dried over MgSO₄ and the solvent was removed by a rotary evaporator, followed by purification using column chromatography, thereby obtaining Compound 7-2 7g (33%).

### Preparation of Compound 52

NaH (60% in mineral oil) 575 mg (14.38 mmol) was diluted in DMF 30 mL. Compound 7-2 2.5 g (11.50 mmol) was dissolved in DMF 20 mL, and the resultant solution was added into the above diluted solution. Then, the mixed solution was stirred at room temperature for 1 hour. 2-Chloro-4,6-diphenyl-1,3,5-triazine 3.0 g (11.50 mmol) was dissolved in DMF 20 Ml, and then this solution was added into the above stirred solution. The resultant mixture was stirred at room temperature for 3 hours, and water 50 mL was added thereinto. The generated solid was filtered under reduced pressure, and the solid thus obtained was recrystallized with DMF and EA, thereby obtaining Compound 52 2.8 g (54 %).
MS/FAB found 515, calculated 514.62

### [Preparation Example 8] Preparation of Compound 71

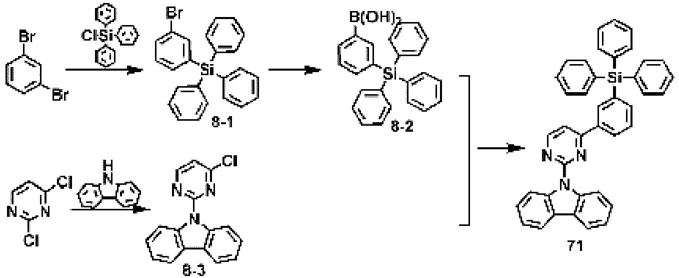

### Preparation of Compound 8-1

1,3-Dibromobenzene 20 g (84.77 mmol) was dissolved in THF 500 mL, and then cooled to a temperature of -78 . n-BuLi 2.5M 33.9 mL(84.77 mmol) was slowly added thereinto, and then stirred at a temperature of -78 for 1 hour. Chlorotriphenylsilane ((C₆H₅)₃SiCl) 29.9 g was dissolved in THF 100 mL, and this solution was added into the above reaction mixture. The resultant mixture was slowly warmed to room temperature, and stirred for 12 hours. Then, extraction with EA, wash with distilled water and aqueous NaCl solution, drying over MgSO₄, distillation under reduced pressure, and recrystallization with MC-MeOH in the ratio of 1:10, were sequentially performed, thereby obtaining Compound **8-1** 18g(95%).

### Preparation of Compound 8-2

Compound **8-1** 20 g (90.06 mmol) was dissolved in THF 600 mL, and then cooled to - 78 . n-BuLi 2.5M 43.2 mL(108.08 mmol) was slowly added thereinto, and then stirred at a temperature of -78 for 1 hour. Trimethylborate 16.06 mL(144.11 mmol) was added thereinto. The resultant mixture was slowly warmed to room temperature, and stirred for 12 hours. Then, extraction with EA, wash with distilled water and aqueous NaCl solution, drying over MgSO₄, distillation under reduced pressure, and recrystallization with MC-MeOH in the ratio of 1:10, were sequentially performed, thereby obtaining Compound 8-2 12 g(35 %).

### Preparation of Compound 8-3

NaH (60% in mineral oil) 3.3 g (83.90 mmol) was diluted in DMF 10 mL. Carbazole 11.2 g(67.12 mmol) was dissolved in DMF 60 mL, and this solution was added into the above reaction solution, and then stirred at room temperature for 1 hour. 2,4-Dichloropyrimidine 10g(67.12mmol) was dissolved in DMF 60 mL, and this solution was added into the above reaction solution. The resultant material was stirred at room temperature for 4 hours, and then distilled water 40 mL was added thereinto. Then, extraction with MC, wash with distilled water and aqueous NaCl solution, drying over MgSO₄, distillation under reduced pressure, and purification using column chromatography were sequentially performed, thereby obtaining Compound **8-3** 4.0 g(21 %).

### Preparation of Compound 71

Compound **8-3** 3.8 g(13.58 mmol), Compound **8-2** 6.2 g(16.30 mmol), Pd(PPh₃)₄ 784 mg (0.67 mmol), 2M aqueous Na₂CO₃ solution 70 mL, EtOH 50 mL and toluene 200 mL was inputted, and then stirred under reflux at 120 for 12 hours. The resultant material was cooled to room temperature, followed by extraction with EA, wash with distilled water and aqueous NaCl solution, and recrystallization with EA, thereby obtaining Compound **71** 5.5 g(69 %).
MS/FAB found 580, calculated 579.76

### [Preparation Example 9] Preparation of Compound 76

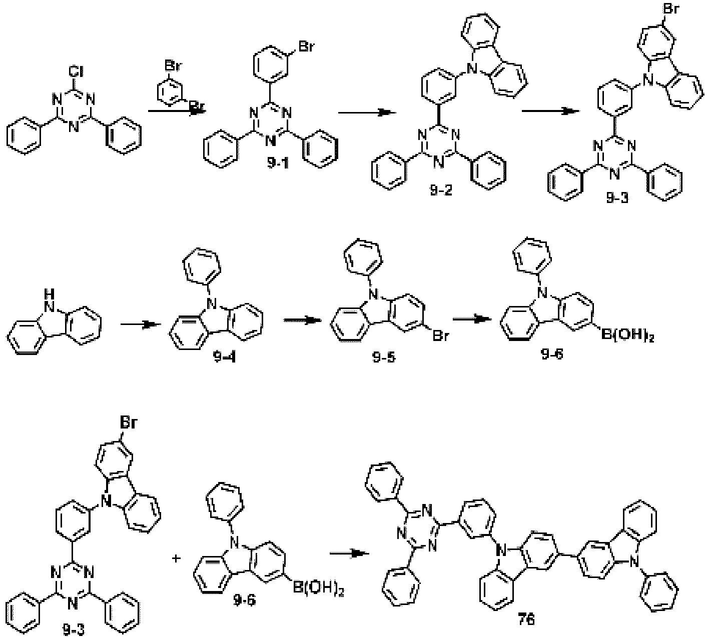

### Preparation of Compound 9-1

1,3-Dibromobenzene 28 g(0.119 mol) was dissolved in THF 600 mL, and then n-BuLi 47.5 mL was slowly added dropwise thereto at -78°C, followed by stirring for 1 hour. 2-Chloro-4,6-diphenyl-1,3,5-triazine 47.5 mL was slowly added dropwise thereto, and the resultant mixture was stirred at room temperature for 5 hours after the temperature was slowly raised. After the reaction is completed, extraction with EA and distilled water and column separation were sequentially performed, thereby obtaining Compound 9-1 15.7 g (40.43 mmol, 40.4%).

### Preparation of Compound 9-2

9H-carbazole 10 g (41.10 mmol) and Compound **9-1** 15.7 g (40.43 mmol) were added into Pd(OAc)₂ 0.46 g, NaOt-bu 7.9 g (82.20 mmol), toluene 100 mL, and P(t-bu)₃ 2 mL (4.11 mmol, 50% in toluene), and stirred under reflux. After 10 hours, the resultant material was cooled to room temperature. Distilled water was put thereinto, followed by extraction with EA, drying over MgSO₄, drying under reduced pressure, and column separation, thereby obtaining Compound **9-2** 12.5 g (26.34 mmol, 65.2%).

### Preparation of Compound 9-3

Compound 9-2 12.5 g (26.34 mmol) was put into a 1-neck flask, which is then filled with argon under vacuum ambient. Tetrahydrofurane 500 mL was put thereinto, followed by stirring at 0 for 10 minutes. NBS 7.35 g (40.78 mmol) was added thereinto, and stirred at room temperature for one day. Upon completion of the reaction, the resultant reaction material was extracted with distilled water and EA. The organic layer was dried over MgSO₄ and the solvent was removed by a rotary evaporator, followed by column chromatography using hexane and EA as a developing solvent, thereby obtaining Compound 9-3 9.8 g (17.71 mmol, 67.3%).

### Preparation of Compound 9-4

9H-carbazole 70 g(0.42 mmol), Iodobenzene 46 mL, Cu 40 g, potassium carbonate 174 g, 18-crown-6 9 g, and 1,2-dichlorobenzene 2 L all are input, and then stirred under reflex for 12 hours. Upon completion of the reaction, extraction with EA, drying over MgSO₄, distillation under reduced pressure, and column separation were sequentially performed, thereby obtaining Compound **9-4** 63.4 g(260.58 mmol, 62%).

### Preparation of Compound 9-5

Compound **9-4** 63.4 g (260.58 mmol) was put into a 1-neck flask, which is then filled with argon under vacuum ambient. Tetrahydrofurane 500 mL was added thereinto, and then stirred at 0 for 10 minutes. NBS 7.35 g (40.78 mmol) was added thereinto, and stirred at room temperature for 1 day. Upon completion of the reaction, the resultant reaction material was extracted with distilled water and EA. The organic layer was dried over MgSO₄ and the solvent was removed by a rotary evaporator, followed by column chromatography using hexane and EA as a developing solvent, thereby obtaining Compound **9-5** 52.4 g (162.63 mmol, 62.4%).

### Preparation of Compound 9-6

Compound **9-5** 52.4 g (162.63 mmol) was put into a 1-neck flask, which is then filled with argon under vacuum ambient. Tetrahydrofurane 500 mL was added thereinto, and then stirred at -78 for 10 minutes. n-BuLi(2.5M in hexane) 15.8 mL (39.45 mmol) was added dropwise thereto, and then stirred at -78 for 1 hour 30 minutes. Trimethylborate 4.85 mL (39.45 mmol) was added thereto at -78 , and then stirred at -78 for 30 minutes followed by at room temperature for 4 hours. Upon completion of the reaction, the resultant reaction material was extracted with distilled water and EA. The organic layer was dried over MgSO₄ and the solvent was removed by a rotary evaporator, followed by column chromatography using hexane and EA as a developing solvent, thereby obtaining Compound **11-6** 20.3 g (70.70 mmol, 43%).

### Preparation of Compound 76

Compound **9-3** 9.8 g (17.71 mmol), Compound **9-6** 20.3 g (70.70 mmol), Pd(PPh₃)₄ 0.8 g (0.7 mmol), 2M aqueous K₂CO₃ solution 20 mL, toluene 100 mL, and ethanol 50 mL were input, and then stirred under reflux for 12 hours. Then, wash with distilled water, extraction with EA, drying over MgSO₄, distillation under reduced pressure, and column separation were sequentially performed, thereby obtaining Compound **76** 5.7 g (7.96 mmol, 50%).
MS/FAB found 716, calculated 715.84

### [Preparation Example 10] Preparation of Compound 77

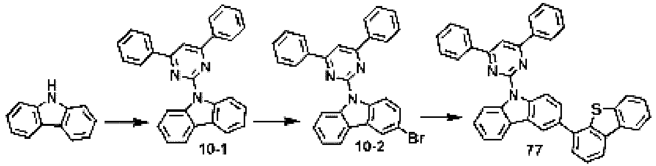

### Preparation of Compound 10-1

NaH 1.57 g (39.36 mmol, 60% in mineral oil) was mixed with DMF 70 mL, and 2-chloro-4,6-diphenylpyrimidine 7 g (26.24 mmol) was dissolved in DMF 60 mL. After 1 hour, 9H-carbazole was dissolved in DMF 70 mL, followed by stirring for 10 hours. Then, addition of water, extraction with EA, drying over MgSO₄, distillation under reduced pressure, and column separation were sequentially performed, thereby obtaining Compound **10-1 7** g(14.78 mmol, 56.33%).

### Preparation of Compound 10-2

Compound **10-1** 7 g (14.78 mmol) was put into a 1-neck flask, which is then filled with argon under vacuum ambient. Tetrahydrofurane 500 mL was added thereinto, and then stirred at 0 for 10 minutes. NBS 7.35 g (40.78 mmol) was added thereinto, and stirred at room temperature for 1 day. Upon completion of the reaction, the resultant reaction material was extracted with distilled water and EA. The organic layer was dried over MgSO₄ and the solvent was removed by a rotary evaporator, followed by column chromatography using hexane and EA as a developing solvent, thereby obtaining Compound **10-2** 5.7 g (11.97 mmol, 80.9%).

### Preparation of Compound 77

Compound **10-2** 5.0 g (17.4 mmol), dibenzo[b,d]thiophen-4-ylboronic acid 5.2 g (20.88 mmol), Pd(PPh₃)₄ 0.8 g (0.7 mmol), 2M aqueous K₂CO₃ solution 20 mL, toluene 100 mL, and ethanol 50 mL were input, and then stirred under reflux for 12 hours. Then, wash with distilled water, extraction with EA, drying over MgSO₄, distillation under reduced pressure, and column separation were sequentially performed, thereby obtaining Compound 77 4.3 g (10.48 mmol, 60%).
MS/FAB found 580, calculated 579.71

### [Preparation Example 11] Preparation of Compound 53

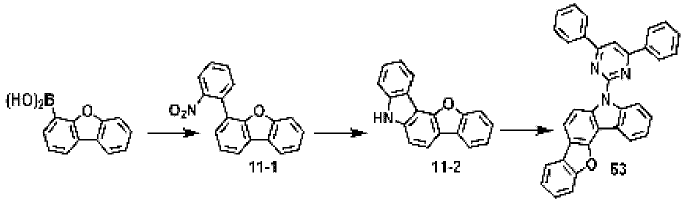

### Preparation of Compound 11-1

Dibenzo[b,d]furan-4-ylboronic acid 10 g (43.84mmol), bromonitrobenzene 8.85g (43.84mmol), 2M aqueous Na₂CO₃ solution 70ml, toluene 200ml, and ethanol 70ml were mixed, and then stirred under reflux. After 5 hours, the resultant material was cooled to room temperature, and then extracted with EA, followed by wash with distilled water. Then, drying over magnesium sulfate, distillation under reduced pressure, and column separation were sequentially performed, thereby obtaining Compound **11-1** 10g (32.74mmol, 74.68%).

### Preparation of Compound 11-2

Compound **11-1** 10g (32.74mmol) was mixed with triethylphosphite 100ml, and then the mixture was stirred at 150 for 7 hours. The resultant material was cooled to room temperature, distilled under reduced pressure, and recrystallized with EA, thereby obtaining Compound **11-2** 7g(25.60mmol, 78.19%).

### Preparation of Compound 53

NaH (60% in mineral oil) 3.3 g (83.90 mmol) was diluted in DMF 10 mL. Compound **11-2** 18.3 g(67.12 mmol) was dissolved in DMF 60 mL, and this solution was added into the above reaction solution, followed by stirring at room temperature for 1 hour. 2-Chloro-4,6-diphenylpyrimidine 10g (67.12mmol)) was dissolved in DMF 60 mL, and this solution was added into the above reaction solution. The resultant material was stirred at room temperature for 4 hours, and then distilled water 40 mL was added thereinto. Then, extraction with MC, wash with distilled water and aqueous NaCl solution, drying over MgSO₄, distillation under reduced pressure, and purification using column chromatography were sequentially performed, thereby obtaining Compound **53** 5.4 g (14.0mmol), 21 %).
MS/FAB found 504, calculated 503.62

### [Preparation Example 12] Preparation of Compound 54

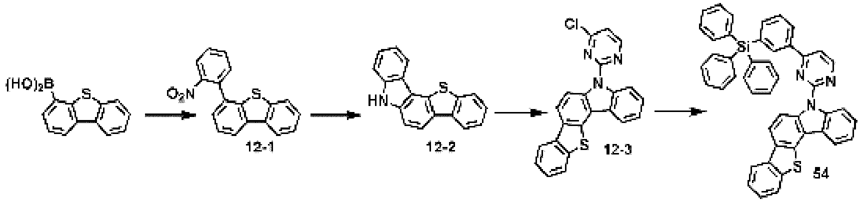

### Preparation of Compound 12-1

Dibenzo[b,d]thiophen-4-ylboronic acid 10 g (43.84mmol), bromonitrobenzene 8.85g (43.84mmol), 2M aqueous Na₂CO₃ solution 70ml, toluene 200ml, and ethanol 70ml were mixed, and then stirred under reflux. After 5 hours, the resultant material was cooled to room temperature, and then extracted with EA, followed by wash with distilled water. Then, drying over magnesium sulfate, distillation under reduced pressure, and column separation were sequentially performed, thereby obtaining Compound 12-1 10g (32.74mmol, 74.68%).

### Preparation of Compound 12-2

Compound **12-1** 10g (32.74mmol) was mixed with triethylphosphite 100ml, and then the mixture was stirred at 150 for 7 hours. The resultant material was cooled to room temperature, distilled under reduced pressure, and recrystallized with EA, thereby obtaining Compound **12-2** 7g (25.60mmol, 78.19%).

### Preparation of Compound 12-3

NaH (60% in mineral oil) 3.3 g (83.90 mmol) was diluted in DMF 10 mL. Compound **12-2** 18.3 g(67.12 mmol) was dissolved in DMF 60 mL, and this solution was added into the above reaction solution, followed by stirring for 1 hour. 2,4-Dichloropyrimidine 10g(67.12mmol) was dissolved in DMF 60 mL, and this solution was added into the above reaction solution. The resultant material was stirred at room temperature for 4 hours, and then distilled water 40 mL was added thereinto. Then, extraction with EA, wash with distilled water and aqueous NaCl solution, drying over MgSO₄, distillation under reduced pressure, and purification using column chromatography were sequentially performed, thereby obtaining Compound **12-3** 5.4 g (14.0mmol), 21 %).

### Preparation of Compound 54

Compound **12-3** 5.2 g(13.58 mmol), Compound **8-2** 6.2 g(16.30 mmol), Pd(PPh₃)₄ 784 mg (0.67 mmol), 2M aqueous Na₂CO₃ solution 70 mL, ethanol 50 mL and toluene 200 mL was inputted, and then stirred under reflux at 120 for 12 hours. The resultant material was cooled to room temperature, followed by extraction with EA, wash with distilled water and aqueous NaCl solution, and recrystallization with EA, thereby obtaining Compound **54** 6.4 g(69 %).
MS/FAB found 686, calculated 685.91

### [Preparation Example 13] Preparation of Compound 58

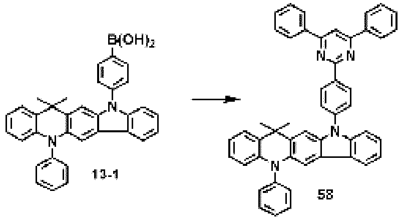

### Preparation of Compound 58

2-Chloro-4,6-diphenylpyrimidine 2.7 g (10.11 mmol), Compound **13-1** 5 g (10.11 mmol), Pd(PPh₃)₄ 584 mg (0.50 mmol), K₂CO₃ (2M) 15 mL and EtOH 15 mL were dissolved in toluene 30 mL, followed by heating at 120 . The resultant material was stirred for 3 hours, and then, upon completion of the reaction, the resultant reaction material was washed with distilled water and then extracted with ethyl acetate. The organic layer was dried over MgSO₄ and the solvent was removed by a rotary evaporator, followed by purification using column chromatography, thereby obtaining Compound **58** 5 g (72%).
MS/FAB found 681, calculated 680.84

### [Preparation Example 14] Preparation of Compound 64

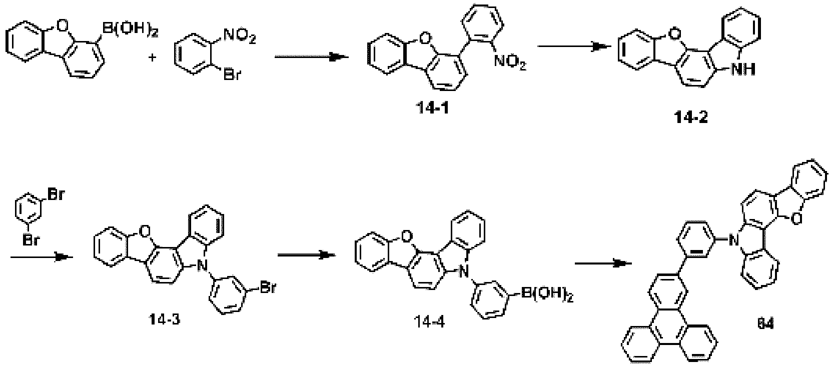

### Preparation of Compound 14-1

Dibenzo[b,d]furan-4-ylboronie acid 45 g (0.21 mol), 1-bromo-2-nitrobenzene 39 g (0.19 mol), Pd(PPh₃)₄ 11.1g (0.0096 mol), K₂CO₃ (2M) 290mL, and ethanol 290 mL were put into a 1L two-neck RBF, and toluene 580mL was added thereinto. The resultant mixture was heated to 120 , and then stirred for 4 hours. Upon completion of the reaction, the resultant material was washed with distilled water, and extracted with ethyl acetate. The organic layer was dried over MgSO₄ and the solvent was removed by a rotary evaporator, followed by purification using column chromatography, thereby obtaining Compound **14-1** 47g (85%).

### Preparation of Compound 14-2

Compound **14-1** 47g (0.16mol), triethylphophite 600mL, and 1,2-dichlorobenzen 300mL were put in a 1L two-neck RBF. The resultant mixture was heated to 150 , and then stirred for 12 hours. Upon completion of the reaction, the solvent was distilled, followed by wash with distilled water and extraction with ethyl acetate. The organic layer was dried over MgSO₄ and the solvent was removed by a rotary evaporator, followed by purification using column chromatography, thereby obtaining Compound **14-2** 39g (81%).

### Preparation of Compound 14-3

Compound **14-2** 15g (0.058mol), 1,3-dibrinibenzene 82g (0.349mol), CuI 5.5g (2.91mmol), K₃PO₄ 25g (0.11mol), ethylene diamine 4mL (0.058mol), and toluene 500mL were put in a 1L two-neck RBF. The resultant mixture was heated to 75 , and then stirred for 12 hours. Upon completion of the reaction, the resultant reaction material was filtered to remove Cu, followed by wash with distilled water and extraction with ethyl acetate. The organic layer was dried over MgSO₄ and the solvent was removed by a rotary evaporator, followed by purification using column chromatography, thereby obtaining Compound 14-3 17.1g(71%).

### Preparation of Compound 14-4

Compound **14-3** 17g (0.041mol) was put in a 1L two-neck RBF, followed by drying under vacuum condition, and then filled with nitrogen gas. THF 300ml was added thereto, and then the resultant mixture was cooled to -78 . n-BuLi(2,5M) 24.7ml (0.061mol) was slowly added thereto, and then stirred for 1 hour while a low-temperature state is maintained. B(i-pro)3 14.2mL (0.061mmol) was added thereto at -78 , followed by stirring for 12 hours. Upon completion of the reaction, 1M HCl was added to the resulting reaction material, and then 10 minutes later, extraction with EA was performed. The organic layer was dried over MgSO₄ and the solvent was removed by a rotary evaporator, followed by purification using column chromatography, thereby obtaining Compound **14-4** 13.8 g (90 %).

### Preparation of Compound 64

2-Bromotriphenylene 7.2g (23.44mmol), Compound **14-4** 13.2g (35.16mmol), Pd(oAc)₂ 790mg (3.51mmol), P(t-Bu)₃ 4.7mL (7.03mmol), and K₃PO₄ (2M) 46mL (93.76mmol) were put in a 500mL two-neck RBF, and ethanol 46mL and toluene 200mL were added thereto. The resultant mixture was heated to 120 , and then stirred for 2 hours. Upon completion of the reaction, the resultant material was washed with distilled water, and extracted with ethyl acetate. The organic layer was dried over MgSO₄ and the solvent was removed by a rotary evaporator, followed by purification using column chromatography, thereby obtaining Compound **64** 5.8g (44%).
MS/FAB found 560, calculated 559.65

### [Preparation Example 15] Preparation of Compound 66

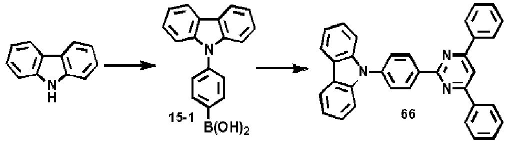

### Preparation of Compound 15-1

Carbazole (30g, 0.18mol), 1,4-dibromobenzene (85g, 0.36mol), CuI (34g, 0.18mol), K₃PO₄ (114g, 0.54mol), and toluene(1200ml) were put into a 500mL round-bottom flask, and the resultant mixture was stirred at 120 for 10 minutes. Then, ethylenediamine (24ml, 0.36mol) was added thereinto, followed by stirring at 120 for 12 hours. After completion of the reaction, extraction with ethyl acetate was performed on the resultant material, followed by column chromatography, thereby obtaining a compound. This compound (13g, 0.04mol) was put into a 1000mL round-bottom flask of anhydrous condition, followed by addition of dried THF (200ml) thereinto, and then n-BuLi (20ml, 2.25M solution in hexane) was slowly added thereinto at -78 while stirring under nitrogen. The resultant mixture was stirred at -78 for 1 hour, followed by slow addition of B(OMe)₃(6.7ml, 0.06mol) thereinto at -78°C, and then the temperature was raised to room temperature, followed by reaction for 12 hours. After completion of the reaction, extraction with ethyl acetate was performed on the resultant material, and then the organic layer is dried over MgSO₄, followed by filtration. The solvent is removed under reduced pressure, followed by column chromatography, thereby obtaining Compound **15-1** (8.5g, 73%) as white solids.

### Preparation of Compound 66

2-chloro-4,6-diphenylpyrimidine (5.0g, 0.02mol), Pd(PPh₃)₄(1.0g, 0.0009mol), 2M Na₂CO₃(100ml), toluene(200ml), and EtOH(70ml) were added into Compound **15-1** (6.4g, 0.02mol), and the resultant mixture was stirred at 120 for 12 hours. After completion of the reaction, extraction with ethyl acetate was performed on the resultant material, and then the organic layer is dried over MgSO₄, followed by filtration. The solvent is removed under reduced pressure, followed by recrystallization with DMF, thereby obtaining Compound 66 (3.7g, 41%).
MS/FAB found 474, calculated 473.57

### [Preparation Example 16] Preparation of Compound 78

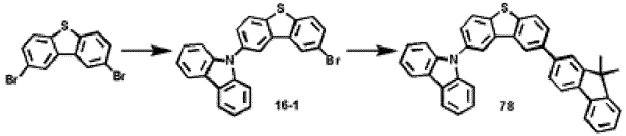

### Preparation of Compound 16-1

2,8-dibromodibenzo[b,d]thiophene 20g (0.12 mol), carbazole 82g (2eq), CuI 11.4g (0.5eq) and K₃PO₄ 76.1g (3eq) were put into a 2-neck flask, which is then filled with nitrogen under vacuum ambient Then, toluene mL(0.1M) was added thereinto, followed by stirring under reflux at 80. When the temperature reached 80 , ethylenediamine 8mL (1eq) was added thereinto, followed by stirring under reflux at 120 for 12 hours. After completion of the reaction, NH₄Cl(aq) 100mL was added thereinto and Cu was removed. The organic layer was extracted with EA and H₂O, followed by drying over MgSO₄, and then the resultant organic layer was distilled under reduced pressure. The organic layer thus obtained was separated by column chromatography, thereby obtaining Compound **16-1** 36g (70% yield).

### Preparation of Compound 78

Compound **16-1** 13.8g (0.03mol), 9,9-dimethyl-9H-fluoren-2-ylboronic acid 11.5g (1.5eq), PdCl₂(PPh₃)₂ 2.3g (1.5eq), 2.5M Na₂CO₃ 15.4g, Aliquat336 5mL, toluene 160mL, EtOH 80 mL, and H₂O 73mL were added, and then stirred under reflux at 110 for 3 hours 30 minutes. After completion of the reaction, extraction with EA and distilled water was performed on the resultant material, followed by drying over MgSO₄, dissolving in CHCl₃, and silica filtration. The organic layer is collected, and then solids were generated by using a rotary evaporator. The generated solids were recrystallized by using DMF, followed by silica filtration of the solids. The organic layer was again used to generate solids by a rotary evaporator, followed by recrystallization with EA and THF, thereby obtaining Compound **78** 8g (49 % yield).
MS/FAB found 542, calculated 541.70

### [Preparation Example 17] Preparation of Compound 95

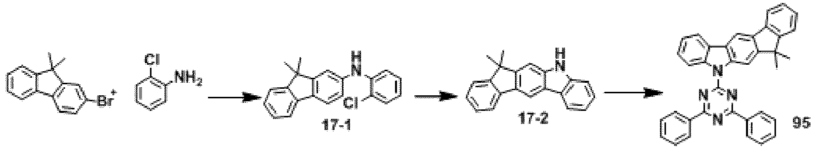

### Preparation of Compound 17-1

2-bromo-9,9-dimethyl-9H-fluorene (50g, 0.183mol), 2-chloroaniline (57ml, 0.549mol), Pd(OAc)₂, (1.6g, 0.007mol), NaO-t-Bu (44g, 0.458mol), toluene(500ml), and P(t-Bu)₃ (7.2ml, 0.0146mol) was put into a 1000mL round-bottom flask, and the resultant mixture was stirred at 120 for 12 hours. After completion of the reaction, extraction with ethyl acetate was performed on the resultant material, and then the organic layer is dried over MgSO₄, followed by filtration. The solvent is removed under reduced pressure, followed by column chromatography, thereby obtaining Compound 17-1 (32g, 55 %) as white solids.

### Preparation of Compound 17-2

Compound **17-1** (32g, 0.1mol), Pd(OAc)₂, (1.1g, 0.005mol), di-tert-butylmethylphosphine.HBF₄ (2.48g, 0.01mol), K₂CO₃ (42g, 0.30mol), and DMA(550ml) were added, and then stirred at 200 for 12 hours. After completion of the reaction, extraction with ethyl acetate was performed on the resultant material, and then the organic layer is dried over MgSO₄, followed by filtration. The solvent is removed under reduced pressure, followed by column chromatography, thereby obtaining Compound **17-2** (14g, 47 %).

### Preparation of Compound 95

Compound **17-2** (5g, 17.64mmol) and 2-Chloro-4,6-diphenyltriazine (5.6g, 21.17mmol) were dissolved in DMF (100ml). NaH (1.05g, 26.46mmol) was slowly added thereinto, followed by stirring at room temperature for 12 hours. Distilled water was added thereinto, and the solids were filtered under reduced pressure, followed by column chromatography, thereby obtaining Compound **95** (3.9g, 42.96%).
MS/FAB found 514, calculated 514.62

### [Preparation Example 18] Preparation of Compound 96

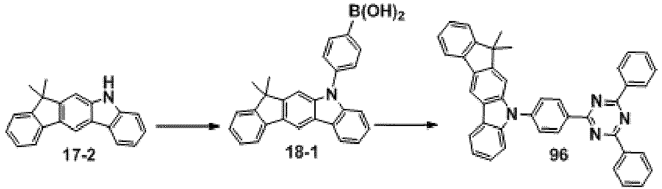

### Preparation of Compound 18-1

Compound **17-2** (32g, 0.11mol), 1-bromo-4-iodobenzene (95.8g, 0.339mol), CuI (13g, 0.068mol), K₃PO₄(86.3g,0.41mol), and toluene(700ml) were put into a 500mL round-bottom flask, and the resultant mixture was stirred at 80 for 10 minutes. Then, ethylenediamine (18.3ml, 0.27mol) was added thereinto, followed by stirring at 140 for 12 hours. After completion of the reaction, extraction with ethyl acetate was performed on the resultant material, followed by column chromatography, thereby obtaining a compound. This compound (46g, 0.10mol) was put into a 2000mL round-bottom flask of anhydrous condition, followed by addition of dried THF (800ml) thereinto, and then n-BuLi (63ml, 2.25M solution in hexane) was added thereinto at -78 while stirring under nitrogen. The resultant mixture was stirred at -78 for 1 hour, followed by slow addition of B(O-iPr)₃(48ml, 0.21mol) thereinto at -78 , and then the temperature was raised to room temperature, followed by reaction for 12 hours. After completion of the reaction, extraction with ethyl acetate was performed on the resultant material, and then the organic layer is dried over MgSO₄, followed by filtration. The solvent is removed under reduced pressure, followed by recrystallization, thereby obtaining Compound **18-1** (32.8g, 78%) as white solids.

### Preparation of Compound 96

Compound **18-1** (32.8g, 0.08mol), 2-chloro-4,6-diphenyl-1,3,5-triazine (26.1g,0.098mol), Pd(PPh₃)₄(4.7g,0.004mol), K₂CO₃(33.7g,0.244mol), toluene(410ml), EtOH(100ml), and H₂O(120ml) were added, and the resultant mixture was stirred at 120 for 12 hours. After completion of the reaction, extraction with ethyl acetate was performed on the resultant material, and then the organic layer is dried over MgSO₄, followed by filtration. The solvent is removed under reduced pressure, followed by recrystallization, thereby obtaining Compound **96** (12g, 30%).
MS/FAB found 591, calculated 590.71

### [Example 1] Manufacture of OLED device using the organic electroluminescent compound according to the present invention

An OLED device was manufactured by using the electroluminescent material of the present invention. First, a transparent electrode ITO thin film (15 Ω/□) obtained from a glass for OLED (manufactured by Samsung Coming) was subjected to ultrasonic washing with trichloroethylene, acetone, ethanol and distilled water, sequentially, and stored in isopropanol before use. Then, the ITO substrate was mounted on a substrate holder of a vacuum deposition apparatus, and N1,N1'-(biphenyl-4,4'-diyl)bis(N1-(naphthalen-2-yl)-N4,N4-diphenylbenzene-1,4-diamine) was put in a cell of the vacuum deposition apparatus, which was then evacuated until vacuum degree in the chamber reached to 10⁻⁶ torr. Then, electric current was applied to the cell to perform vaporization, thereby forming a hole injection layer having a thickness of 120 nm on the ITO substrate. Then, N4,N4,N4',N4'-tetra(biphenyl-4-yl)biphenyl-4,4'-diamine was put in another cell of the vacuum vapor deposition apparatus, and electric current was applied to the cell to perform vaporization, thereby forming a hole transport layer having a thickness of 20 nm on the hole injection layer. After forming the hole injection layer and the hole transport layer, an electroluminescent layer was formed thereon as follows. Compound **53** as a host was put in a cell and Compound **1** as a dopant was put in another cell, within a vacuum vapor deposition apparatus. The two materials were vaporized at different rates to perform doping of below 20 wt%, thereby forming an electroluminescent layer having a thickness of 40 nm on the hole transport layer. Subsequently, 2-(4-(9,10-di(naphthalen-2-yl)anthracen-2-yl)phenyl)-1-phenyl-1H-benzo[d]imidazole) was put in put in a cell of the vacuum deposition apparatus, and Lithium quinolate was put in another cell of the vacuum deposition apparatus. Then the two materials were vaporized at different rates to perform doping of 30 wt% to 70 wt%, thereby forming an electron transfer layer having a thickness of 30 nm on the electroluminescent layer. Next, lithium quinolate was deposited to have a thickness of 1 to 2 nm as an electron injection layer, and then an Al cathode is deposited to have a thickness of 150nm by using another vacuum deposition apparatus, thereby manufacturing an OLED device. Respective compounds according to the materials were purified by vacuum sublimation under 10-6 torr.

As a result, a current of 3.0mA/cm² flowed at a voltage of 4.0V, and it was confirmed that green light of 2038 cd/m² was emitted.

### [Example 2] Manufacture of OLED device using the organic electroluminescent compound according to the present invention

An OLED device was manufactured by the same method as Example 1 except that, as electroluminescent materials, Compound 54 was used as a host.

As a result, a current of 2.0mA/cm² flowed at a voltage of 3.6V, and it was confirmed that green light of 1035 cd/m² was emitted.

### [Example 3] Manufacture of OLED device using the organic electroluminescent compound according to the present invention

An OLED device was manufactured by the same method as Example 1 except that, as electroluminescent materials, Compound **59** was used as a host.

As a result, a current of 1.56mA/cm² flowed at a voltage of 3.7V, and it was confirmed that green light of 1020 cd/m² was emitted. It took 40 hours to decrease luminescence by 90% at brightness of 15000nit.

### [Example 4] Manufacture of OLED device using the organic electroluminescent compound according to the present invention

An OLED device was manufactured by the same method as Example 1 except that, as electroluminescent materials, Compound **62** was used as a host.

As a result, a current of 1.91mA/cm² flowed at a voltage of 3.7V, and it was confirmed that green light of 1105 cd/m² was emitted.

### [Example 5] Manufacture of OLED device using the organic electroluminescent compound according to the present invention

An OLED device was manufactured by the same method as Example 1 except that, as electroluminescent materials, Compound 63 was used as a host.

As a result, a current of 1.9mA/cm² flowed at a voltage of 3.0V, and it was confirmed that green light of 1070 cd/m² was emitted.

### [Example 6] Manufacture of OLED device using the organic electroluminescent compound according to the present invention

An OLED device was manufactured by the same method as Example 1 except that, as electroluminescent materials, Compounds **63 and 48** were evaporated at the same speed and used as a host.

As a result, a current of 1.73mA/cm² flowed at a voltage of 3.0V, and it was confirmed that green light of 760 cd/m² was emitted. It took 35 hours to decrease luminescence by 90% at brightness of 15000nit.

### [Example 7] Manufacture of OLED device using the organic electroluminescent compound according to the present invention

An OLED device was manufactured by the same method as Example 1 except that, as electroluminescent materials, Compound **65** was used as a host.

As a result, a current of 2.3mA/cm² flowed at a voltage of 3.4V, and it was confirmed that green light of 1220 cd/m² was emitted.

### [Example 8] Manufacture of OLED device using the organic electroluminescent compound according to the present invention

An OLED device was manufactured by the same method as Example 1 except that, as electroluminescent materials, Compound **66** was used as a host.

As a result, a current of 3.2mA/cm² flowed at a voltage of 4.0V, and it was confirmed that green light of 1760 cd/m² was emitted. It took 32 hours to decrease luminescence by 90% at brightness of 15000nit.

### [Example 9] Manufacture of OLED device using the organic electroluminescent compound according to the present invention

An OLED device was manufactured by the same method as Example 1 except that, as electroluminescent materials, Compound **71** was used as a host.

As a result, a current of 2.2mA/cm² flowed at a voltage of 4.1V, and it was confirmed that green light of 1030 cd/m² was emitted.

### [Example 10] Manufacture of OLED device using the organic electroluminescent compound according to the present invention

An OLED device was manufactured by the same method as Example 1 except that, as electroluminescent materials, Compound 77 was used as a host.

As a result, a current of 2.08mA/cm² flowed at a voltage of 3.7V, and it was confirmed that green light of 1020 cd/m² was emitted.

### [Example 11] Manufacture of OLED device using the organic electroluminescent compound according to the present invention

An OLED device was manufactured by the same method as Example 1 except that, as electroluminescent materials, Compounds **78 and 48** were evaporated at the same speed and used as a host.

As a result, a current of 1.6mA/cm² flowed at a voltage of 4.3V, and it was confirmed that green light of 820 cd/m² was emitted. It took 33 hours to decrease luminescence by 90% at brightness of 15000nit.

### [Example 12] Manufacture of OLED device using the organic electroluminescent compound according to the present invention

An OLED device was manufactured by the same method as Example 1 except that, as electroluminescent materials, Compound **94** was used as a host.

As a result, a current of 1.92mA/cm² flowed at a voltage of 3.8V, and it was confirmed that green light of 1060 cd/m² was emitted.

### [Example 13] Manufacture of OLED device using the organic electroluminescent compound according to the present invention

An OLED device was manufactured by the same method as Example 1 except that, as electroluminescent materials, Compound **95** was used as a host.

As a result, a current of 2.81mA/cm² flowed at a voltage of 3.3V, and it was confirmed that green light of 1315 cd/m² was emitted.

### [Example 14] Manufacture of OLED device using the organic electroluminescent compound according to the present invention

An OLED device was manufactured by the same method as Example 1 except that, as electroluminescent materials, Compound **96** was used as a host.

As a result, a current of 2.49mA/cm² flowed at a voltage of 2.8V, and it was confirmed that green light of 860 cd/m² was emitted.

### [Example 15] Manufacture of OLED device using the organic electroluminescent compound according to the present invention

An OLED device was manufactured by the same method as Example 1 except that, as electroluminescent materials, Compound 97 was used as a host.

As a result, a current of 2.1mA/cm² flowed at a voltage of 3.5V, and it was confirmed that green light of 1018 cd/m² was emitted.

### [Comparative example 1] Manufacture of OLED device using an electroluminescent material of the prior art.

An OLED device was manufactured by the same method as Example 1 except that, an electroluminescent layer having a thickness of 30 nm is deposited on a hole transfer layer by using CBP[4,4'-N,N'-dicarbazole-biphenyl] as a host and Ir(ppy)3[tris(2-phenylpyridine)iridium as a dopant, as electroluminescent materials, and a hole blocking layer having a thickness of 10 nm is deposited by using BAlq[bis(2-methyl-8-quinolinate)(p-phenylphenolato) aluminum **(III)**.

As a result, a current of 5.0mA/cm² flowed at a voltage of 6.0V, and it was confirmed that green light of 1183 cd/m² was emitted. It took 0.5 hours to decrease luminescence by 90% at brightness of 15000nit.

### [Comparative example 2] Manufacture of OLED device using an electroluminescent material of the prior art.

An OLED device was manufactured by the same method as Example 1 except that, an electroluminescent layer having a thickness of 30 nm is deposited on a hole transfer layer by using Compound **66** as a host and Ir(ppy)3 [tris(2-phenylpyridine)iridium] as a dopant, as electroluminescent materials.

As a result, a current of 1.89mA/cm² flowed at a voltage of 4.6V, and it was confirmed that green light of 920 cd/m² was emitted. It took 11 hours to decrease luminescence by 90% at brightness of 15000nit.

### [Comparative example 3] Manufacture of OLED device using an electroluminescent material of the prior art.

An OLED device was manufactured by the same method as Example 1 except that, an electroluminescent layer having a thickness of 30 nm is deposited on a hole transfer layer by using Compound **66** as a host and Compound 30 as a dopant, as electroluminescent materials.

As a result, a current of 2.0mA/cm² flowed at a voltage of 4.4V, and it was confirmed that green light of 1120 cd/m² was emitted. It took 28 hours to decrease luminescence by 90% at brightness of 15000nit.

The test data confirmed that when the specific host according to an exemplary embodiment and tris(4-methyl-2,5-diphenylpyridine)Iridium were used together on the electroluminescent layer, excellent luminescent efficiency and long operation life were exhibited. In addition, the dopant according to an exemplary embodiment showed excellent properties when the dopant was used with two hosts.

## Claims

1. An organic electroluminescent device in which an organic layer is interposed between an anode and a cathode on a substrate, wherein the organic layer comprises an electroluminescent layer containing one or more dopant compounds represented by Chemical Formula 1 below and one or more host compounds represented by Chemical Formulas 2 to 5 below. 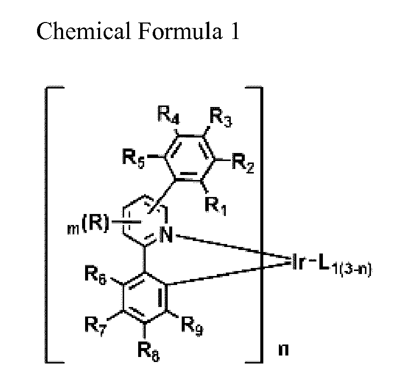 [wherein
L₁ represents an organic ligand;
R represents hydrogen, substituted or unsubstituted (C1-C30)alkyl, substituted or unsubstituted (C1-C30)alkoxy, substituted or unsubstituted (C6-C30)aryl or substituted or unsubstituted (C3-C30)heteroaryl;
R₁ through R₅ independently represent hydrogen, deuterium, halogen, substituted or unsubstituted (C3-C30)cycloalkyl, substituted or unsubstituted 5- to 7-membered heterocycloalkyl, cyano, nitro, BR₁₁R₁₂, PR₁₃R₁₄, P(=O)R₁₅R₁₆, R₁₇R₁₈R₁₉Si-, or substituted or unsubstituted (C6-C30)ar(C1-C30)alkyl;
R₆ through R₉ represent hydrogen, deuterium, halogen, substituted or unsubstituted (C1-C30)alkyl, substituted or unsubstituted (C1-C30)aryl, substituted or unsubstituted (C5-C30)heteroaryl, substituted or unsubstituted (C3-C30)cycloalkyl, substituted or unsubstituted 5- to 7-membered heterocycloalkyl, cyano, nitro, BR₁₁R₁₂, PR₁₃R₁₄, P(=O)R₁₅R₁₆, R₁₇R₁₈R₁₉Si-, NR₂₀R₂₁, R₂₂Y-, substituted or unsubstituted (C2-C30)alkenyl, substituted or unsubstituted (C2-C30)alkynyl, substituted or unsubstituted (C6-C30)ar(C1-C30)alkyl or they are linked to adjacent substituents to form a fused ring;
R₁₁ through R₂₂ independently represent substituted or unsubstituted (C1-C30)alkyl, substituted or unsubstituted (C6-C30)aryl or substituted or unsubstituted (C3-C30)heteroaryl;
Y represents S or O;
n and m independently represent an integer of 1 to 3;
the heterocycloalkyl and heteroaryl include one or more hetero atoms selected from the group consisting of B, N, O, S, P(=O), Si and P.] 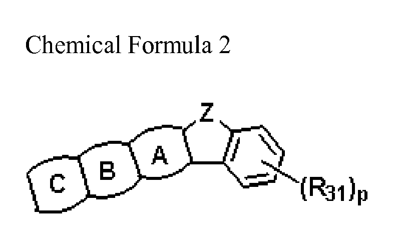 [wherein
Z represents -O-, -S-, -C(R₄₁R₄₂)-, -Si(R₄₃R₄₄)- or -N(R₄₅)-;
a ring A and a ring C independently represent 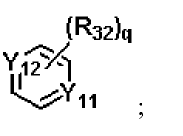 a ring B represents a ring of 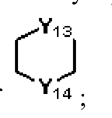 Y₁₁ through Y₁₂ independently represent C and N;
Y₁₃ through Y₁₄ independently represent a chemical bond, -O-, -S-, -C(R₄₁R₄₂)-, - Si(R₄₃R₄₄)- or -N(R₄₅)-; only except for the case where Y₁₃ and Y₁₄ represent a chemical bond at the same time;
R₃₁ and R₃₂ independently represent hydrogen, deuterium, halogen, substituted or unsubstituted (C1-C30)alkyl, substituted or unsubstituted (C6-C30)aryl, substituted or unsubstituted (C3-C30)heteroaryl, substituted or unsubstituted (C3-C30)cycloalkyl, substituted or unsubstituted 5- to 7-membered heterocycloalkyl, substituted or unsubstituted (C6-C30)ar(C1-C30)alkyl, substituted or unsubstituted (C1-C30)alkylsilyl group, substituted or unsubstituted (C1-C30)arylsilyl group, substituted or unsubstituted (C1-C30)alkyl(C6-C30)arylsilyl group, cyano, nitro, or hydroxyl, or they are linked to an adjacent substituent via substituted or unsubstituted (C3-C30)alkylene or (C3-C30)alkenylene with or without a fused ring to form an alicyclic ring and a monocyclic or polycyclic aromatic ring;
the R₄₁ through R₄₅ independently represent hydrogen, deuterium, halogen, substituted or unsubstituted (C1-C30)alkyl, substituted or unsubstituted (C6-C30)aryl, substituted or unsubstituted (C3-C30)heteroaryl, substituted or unsubstituted 5- to 7-membered heterocycloalkyl, substituted or unsubstituted (C3-C30)cycloalkyl or they are linked to adjacent substituents to form a ring;
p and q independently represent an integer of 0 to 4;
when p or q represent an integer larger than 2, each R₃₁ and R₃₂ may be the same or different from each other, and they may be linked to adjacent substituents to form a ring; and
the heterocycloalkyl and heteroaryl include one or more hetero atoms selected from the group consisting of B, N, O, S, P(=O), Si and P.]
Chemical Formula 3 **(Cz-L₂)ₐ-M**
Chemical Formula 4 **(Cz)_{b}-L₂-M**
[wherein
Cz is selected from following structures, 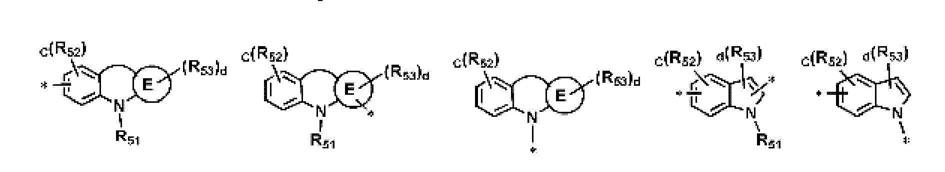 a ring E represents a (C6-C30)cycloalkyl group, a (C6-C30)aryl group, or a (C5-C30)heteroaryl group;
R₅₁ through R₅₃ independently represent hydrogen, deuterium, halogen, substituted or unsubstituted (C1-C30)alkyl, substituted or unsubstituted (C6-C30)aryl, substituted or unsubstituted (C3-C30)heteroaryl, substituted or unsubstituted 5- to 7-membered heterocycloalkyl, substituted or unsubstituted (C6-C30)aryl fused with one or more substituted or unsubstituted (C3-C30)cycloalkyl, 5- to 7-membered heterocycloalkyl fused with one or more substituted or unsubstituted aromatic rings, substituted or unsubstituted (C3-C30)cycloalkyl, (C3-C30)cycloalkyl fused with one or more substituted or unsubstituted aromatic rings, substituted or unsubstituted (C6-C30)ar(C1-C30)alkyl, cyano, nitro, hydroxyl, BR₁₁R₁₂, PR₁₃R₁₄, P(=O)R₁₅R₁₆, R₁₇R₁₈R₁₉Si-, NR₂₀R₂₁, -YR₂₂ or they are linked to an adjacent substituent via substituted or unsubstituted (C3-C30)alkylene or substituted or unsubstituted (C3-C30)alkenylene with or without a fused ring to form an alicyclic ring and a monocyclic or polycyclic aromatic ring, carbon atoms of the formed alicyclic ring and monocyclic or polycyclic aromatic ring may be substituted with one or more hetero atoms selected from the group consisting of nitrogen, oxygen and sulfur; and
each R₅₂ or R₅₃ may be the same or different from each other;
L₂ represents a chemical bond, a substituted or unsubstituted (C6-C30)aryl group, or a substituted or unsubstituted (C5-C30)heteroaryl group;
M represents a substituted or unsubstituted (C6-C30)aryl group, or substituted or unsubstituted (C5-C30)heteroaryl;
a through d independently represent an integer of 0 to 4.] 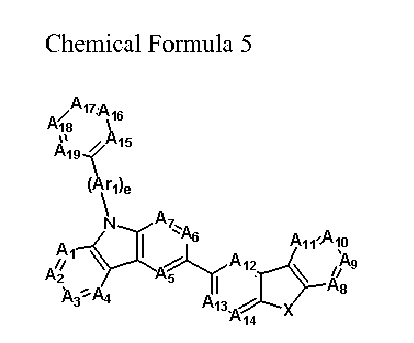 [wherein
A₁ through A₁₉ independently represent CR₆₁ or N;
X represents -C(R₆₂R₆₃)-, -N(R₆₄)-, -S-, -O-, -Si(R₆₅)(R₆₆)-, -P(R₆₇)-, -P(=O)(R₆₈)- or - B(R₆₉)-;
Ar₁ represents substituted or unsubstituted (C6-C40)arylene, or substituted or unsubstituted (C3-C40)heteroarylene; only except for the case where e=0 and A₁₅ through A₁₉ are CR₆₁ at the same time,
R₆₁ through R₆₉ independently represent hydrogen, deuterium, halogen, substituted or unsubstituted (C1-C30)alkyl, substituted or unsubstituted (C6-C30)aryl, substituted or unsubstituted (C6-C30)aryl fused with one or more substituted or unsubstituted (C3-C30)cycloalkyl, substituted or unsubstituted (C3-C30)heteroaryl, substituted or unsubstituted 5- to 7-membered heterocycloalkyl, 5- to 7-membered heterocycloalkyl fused with one or more substituted or unsubstituted aromatic rings, substituted or unsubstituted (C3-C30)cycloalkyl, substituted or unsubstituted fused with one or more aromatic rings (C3-C30)cycloalkyl, cyano, trifluoromethyl, NR₇₁R₇₂, BR₇₃R₇₄, PR₇₅R₇₆, P(=O)R₇₇R₇₈, R₇₉R₈₀R₈₁Si-, R₈₂Y₂₁-, R₈₃C(=O)-, R₈₄C(=O)O-, substituted or unsubstituted (C6-C30)ar(C1-C30)alkyl, substituted or unsubstituted (C2-C30)alkenyl, substituted or unsubstituted (C2-C30)alkynyl, carboxyl, nitro, or hydroxyl, or they are linked to an adjacent substituent via substituted or unsubstituted (C3-C30)alkylene or substituted or unsubstituted (C3-C30)alkenylene with or without a fused ring to form an alicyclic ring, a monocyclic or polycyclic aromatic ring, or a hetero aromatic ring;
the heterocycloalkyl and heteroaryl include one or more hetero atoms selected from the group consisting of B, N, O, S, P(=O), Si and P;
the R₇₁ through R₇₈ independently represent substituted or unsubstituted (C1-C30)alkyl, substituted or unsubstituted (C6-C30)aryl or substituted or unsubstituted (C3-C30)heteroaryl,
the R₇₉ through R₈₁ independently represent substituted or unsubstituted (C1-C30)alkyl or substituted or unsubstituted (C6-C30)aryl,
the Y₂₁ represents S or O,
R₈₂ represents substituted or unsubstituted (C1-C30)alkyl or substituted or unsubstituted (C6-C30)aryl,
the R₈₃ represents substituted or unsubstituted (C1-C30)alkyl, substituted or unsubstituted (C1-C30)alkoxy, substituted or unsubstituted (C6-C30)aryl or substituted or unsubstituted (C6-C30)aryloxy,
the R₈₄ represents substituted or unsubstituted (C1-C30)alkyl, substituted or unsubstituted (C1-C30)alkoxy, substituted or unsubstituted (C6-C30)aryl or substituted or unsubstituted (C6-C30)aryloxy, and
e represents an integer of 0 or 2.]

2. The organic electroluminescent device of claim 1, wherein a substituent further substituted with the R, R₁ through R₉, R₁₁ through R₁₂, R₃₁ through R₃₂, R₄₁ through R₄₅, R₅₁ through R₅₃, R₆₁ through R₆₉, R₇, through R₈₄, L₂, M and Ar₁ independently represent one or more selected from the group consisting of deuterium, halogen, halogen-substituted or unsubstituted (C1-C30)alkyl, (C6-C30)aryl, (C6-C30)aryl-substituted or unsubstituted (C3-C30)heteroaryl, 5- to 7-membered heterocycloalkyl, 5- to 7-membered heterocycloalkyl fused with one or more aromatic rings, (C3-C30)cycloalkyl, (C6-C30)cycloalkyl fused with one or more aromatic rings, R₉₁R₉₂R₉₃Si-, (C2-C30)alkenyl, (C2-C30)alkynyl, cyano, carbazolyl, NR₉₄R₉₅, BR₉₆R₉₇, PR₉₈R₉₉, P(=O)R₁₀₀R₁₀₁, (C6-C30)ar(C1-C30)alkyl, (C1-C30)alkyl(C6-C30)aryl, R₁₀₂S-, R₁₀₃O-, R₁₀₄C(=O)-, R₁₀₅C(=O)O-, carboxyl, nitro or hydroxyl,
R₉₁ through R₁₀₃ independently represent hydrogen, deuterium, halogen, substituted or unsubstituted (C1-C30)alkyl, substituted or unsubstituted (C6-C30)aryl, substituted or unsubstituted (C3-C30)heteroaryl, or substituted or unsubstituted 5- to 7-membered heterocycloalkyl or they are linked to an adjacent substituent via substituted or unsubstituted (C3-C30)alkylene or substituted or unsubstituted (C3-C30)alkenylene with or without a fused ring to form an alicyclic ring and a monocyclic or polycyclic aromatic ring, carbon atoms of the formed alicyclic ring and monocyclic or polycyclic aromatic ring may be substituted with one or more hetero atoms selected from the group consisting of nitrogen, oxygen and sulfur; and
R₁₀₄ and R₁₀₅ represent (C1-C30)alkyl, (C1-C30)alkoxy, (C6-C30)aryl or (C6-C30)aryloxy.

3. The organic electroluminescent device of claim 1, wherein Chemical Formula 1 is represented by Chemical Formulas 6 to 7; Chemical Formula 2 is represented by Chemical Formulas 8 to 13; and Cz of Chemical Formulas 3 to 4 is represented by following structures. 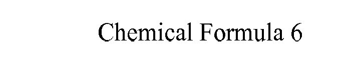 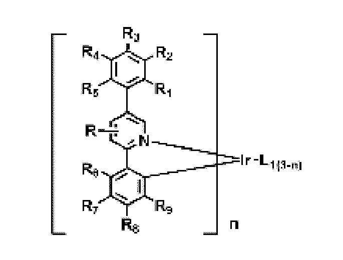 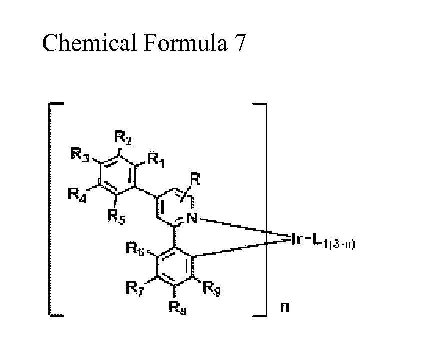 [wherein
R, R₁ through R₉, L₁ and n are the same as defined in Chemical Formula 1.] 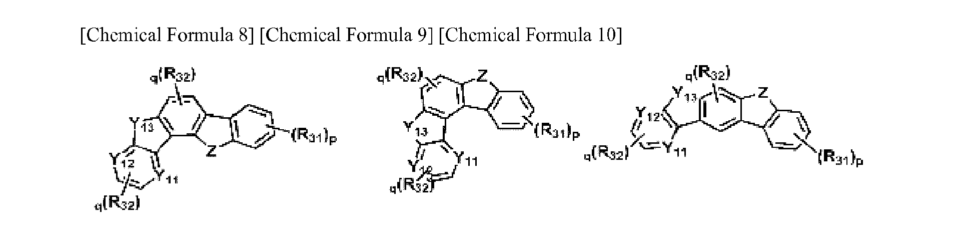 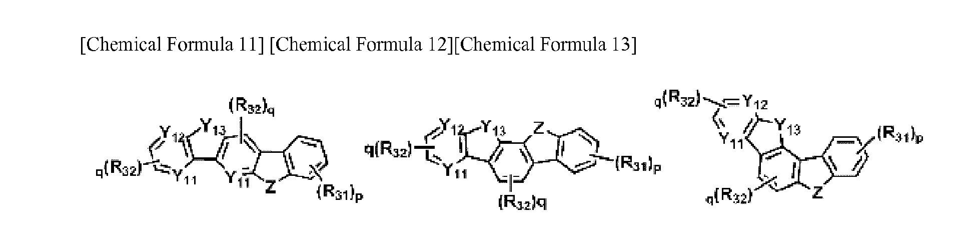 [wherein
R₃₁, R₃₂, Y₁₁ through Y₁₃, Z, p and q are the same as defined in Chemical Formula 2.] wherein Cz of Chemical Formulas 3 and 4 is selected from following structures, and 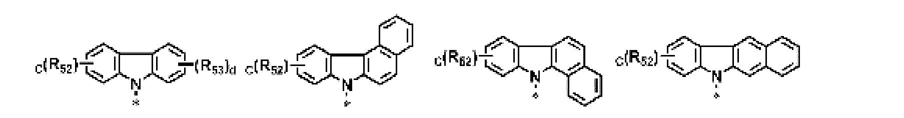 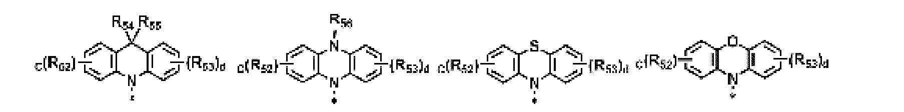 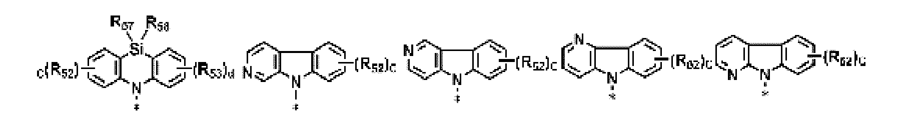 [wherein
R₅₂, R₅₃, c and d are the same as defined in Chemical Formulas 3 and 4; and R₅₄ through R₅₈ independently represent halogen, substituted or unsubstituted (C1-C30)alkyl, substituted or unsubstituted (C6-C30)aryl, or substituted or unsubstituted (C3-C30)heteroaryl or carbazolyl.]

4. The organic electroluminescent device of claim 1, wherein L₁ is represented by Chemical Formula 1 is selected from following structures. 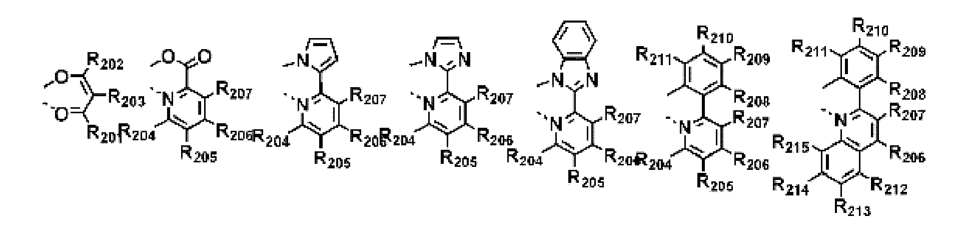 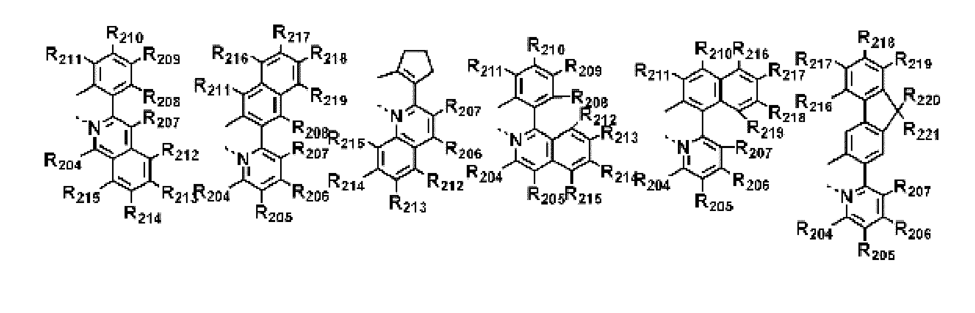 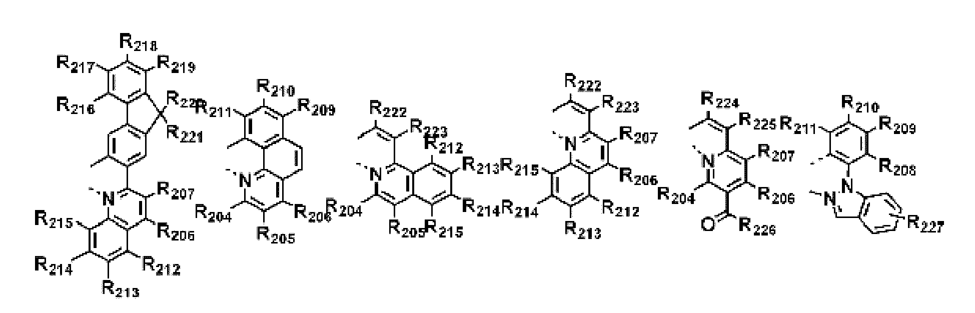 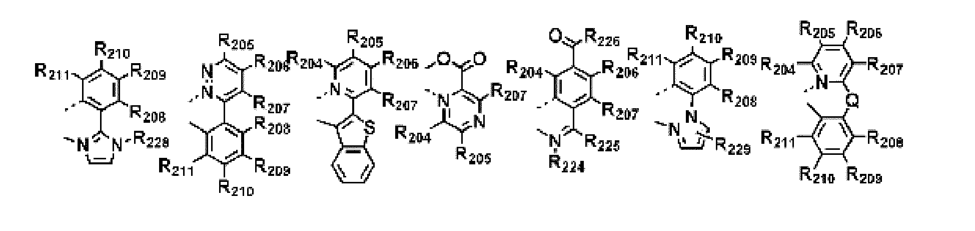 [wherein
the R₂₀₁ through R₂₀₃ independently represent hydrogen, deuterium, halogen-substituted or unsubstituted (C1-C30)alkyl, (C1-C30)alkyl-substituted or unsubstituted (C6-C30)aryl or halogen;
R₂₀₄ through R₂₁₉ independently represent hydrogen, deuterium, substituted or unsubstituted (C1-C30)alkyl, substituted or unsubstituted (C1-C30)alkoxy, substituted or unsubstituted (C3-C30)cycloalkyl, substituted or unsubstituted (C2-C30)alkenyl, substituted or unsubstituted (C6-C30)aryl, substituted or unsubstituted mono- or substituted or unsubstituted di-(C1-C30)alkylamino, substituted or unsubstituted mono or di-(C6-C30)arylamino, SF₅, substituted or unsubstituted tri(C1-C30)alkylsilyl, substituted or unsubstituted di(C1-C30)alkyl(C6-C30)arylsilyl, substituted or unsubstituted tri(C6-C30)arylsilyl, cyano or halogen;
R₂₂₀ through R₂₂₃ independently represent hydrogen, deuterium, halogen-substituted or unsubstituted (C1-C30)alkyl or (C1-C30)alkyl-substituted or unsubstituted (C6-C30)aryl;
R₂₂₄ and R₂₂₅ independently represent hydrogen, deuterium, substituted or unsubstituted (C1-C30)alkyl, substituted or unsubstituted (C6-C30)aryl or halogen, or R₂₂₄ and R₂₂₅ are linked via (C3-C12)alkylene or (C3-C12)alkenylene with or without a fused ring to form an alicyclic ring and a monocyclic or polycyclic aromatic ring;
R₂₂₆ represents substituted or unsubstituted (C1-C30)alkyl, substituted or unsubstituted (C6-C30)aryl, substituted or unsubstituted (C5-C30)heteroaryl or halogen;
R₂₂₇ through R₂₂₉ independently represent hydrogen, deuterium, substituted or unsubstituted (C1-C30)alkyl, substituted or unsubstituted (C6-C30)aryl or halogen;
Q represents 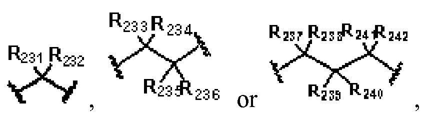 and
R₂₃₁ through R₂₄₂ independently represent hydrogen, deuterium, halogen-substituted or unsubstituted (C1-C30)alkyl, (C1-C30)alkoxy, halogen, substituted or unsubstituted (C6-C30)aryl, cyano, substituted or unsubstituted (C5-C30)cycloalkyl, or they are linked to an adjacent substituent via alkylene or alkenylene to form a spiro ring or a fused ring, or linked to R₂₀₇ or R₂₀₈ via alkylene or alkenylene to form a saturated or unsaturated fused ring.]

5. The organic electroluminescent device of claim 1, wherein the Chemical Formula 1 is represented by following Chemical Formula 14 and the Chemical Formulas 2 to 5 are represented by following Chemical Formulas 8, 10, 11 to 12 and Chemical Formulas 3 to 5: 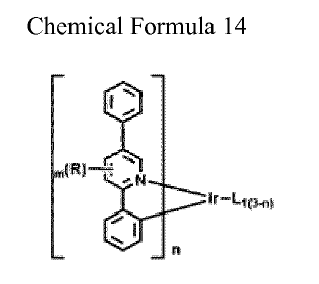 wherein
R represents substituted or unsubstituted (C1-C30)alkyl, substituted or unsubstituted (C6-C30)aryl;
L₁ is selected from the following structures, and 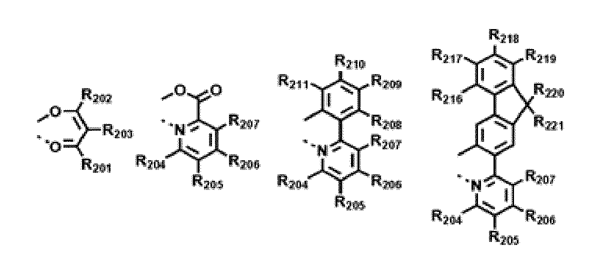 the R₂₀, through R₂₀₃ independently represent hydrogen, deuterium, halogen-substituted or unsubstituted (C1-C30)alkyl, (C1-C30)alkyl-substituted or unsubstituted (C6-C30)aryl or halogen;
R₂₀₄ through R₂₁₉ independently represent hydrogen, deuterium, substituted or unsubstituted (C1-C30)alkyl, substituted or unsubstituted (C6-C30)aryl, SF₅, substituted or unsubstituted tri(C1-C30)alkylsilyl, substituted or unsubstituted di(C1-C30)alkyl(C6-C30)arylsilyl, substituted or unsubstituted tri(C6-C30)arylsilyl, cyano or halogen;
R₂₂₀ through R₂₂₁ independently represent hydrogen, deuterium, halogen-substituted or unsubstituted (C1-C30)alkyl or (C1-C30)alkyl-substituted or unsubstituted (C6-C30)aryl, and
n, and m independently represent an integer of 1 to 3. 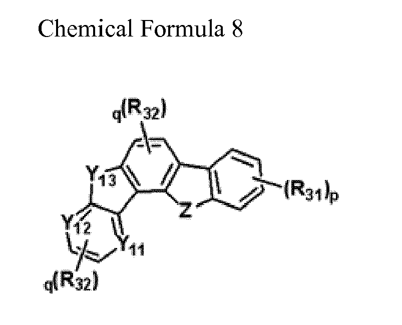 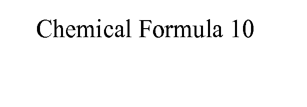 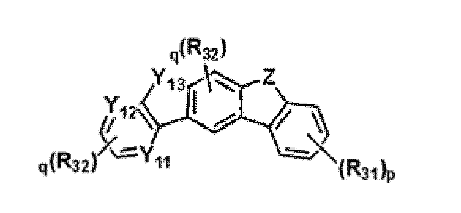 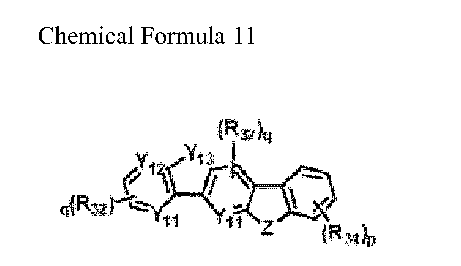 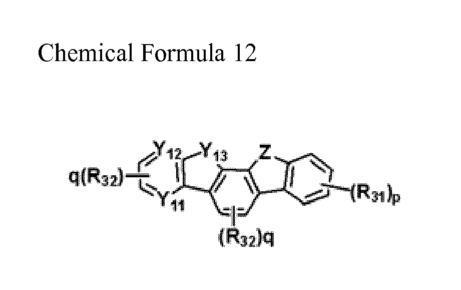 wherein
Z represents -O-, -S-, -C(R₄₁R₄₂)-, -N(R₄₅)-;
Y₁₁ through Y₁₂ represent C;
Y₁₃ represents -N(R₄₅)-;
R₃₁ and R₃₂ independently represent hydrogen, deuterium, halogen, substituted or unsubstituted (C1-C30)alkyl, substituted or unsubstituted (C6-C30)aryl, substituted or unsubstituted (C3-C30)heteroaryl, substituted (C1-C30)alkylsilyl group, substituted or unsubstituted (C1-C30)arylsilyl group, and substituted or unsubstituted (C1-C30)alkyl(C6-C30)arylsilyl group,
the R₄₁ through R₄₂ independently represent substituted or unsubstituted (C1-C30)alkyl, substituted or unsubstituted (C6-C30)aryl, and substituted or unsubstituted (C3-C30)heteroaryl,
the R₄₅ represents unsubstituted (C1-C30)alkyl, substituted or unsubstituted (C6-C30)aryl, and substituted or unsubstituted (C3-C30)heteroaryl,
p and q independently represent an integer of 0 through 4; when p or q represent an integer larger than 2, each R₃₁ and R₃₂ may be the same or different from each other.
Chemical Formula 3
(Cz-L₂)ₐ-M
Chemical Formula 4
(Cz)_{b}-L₂-M
wherein
Cz has a following structure, 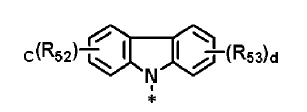 R₅₂ through R₅₃ independently represent hydrogen, deuterium, halogen, substituted or unsubstituted (C1-C30)alkyl, substituted or unsubstituted (C6-C30)aryl, substituted or unsubstituted (C3-C30)heteroaryl, R₁₇R₁₈R₁₉Si-,, R₁₇ through R₁₉ independently represent substituted or unsubstituted (C1-C30)alkyl, substituted or unsubstituted (C6-C30)aryl; each R₅₂ or R₅₃ may be the same or different from each other;
L₂ represents a chemical bond, substituted or unsubstituted (C6-C30)arylene, and substituted or unsubstituted (C5-C30)heteroarylene;
M represents substituted or unsubstituted (C6-C30)aryl group, and substituted or unsubstituted (C5-C30)heteroaryl;
a through d independently represent an integer of 0 through 4. 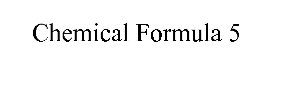 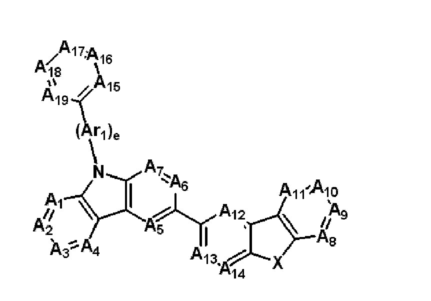 wherein
A₁ through A₁₄ represent CR₆₁;
A15 through A19 independently represent CR₆₁ or N,
X represents -N(R₆₄)-, -S-, -O-, and -Si(R₆₅)(R₆₆)-;
Ar₁ represents substituted or unsubstituted (C6-C40)arylene, and substituted or unsubstituted (C3-C40)heteroarylene; except for the case that e=0 and A₁₅ through A₁₉ represent CR₆₁ at the same time,
R₆₁ and R₆₄ through R₆₆ independently represent hydrogen, deuterium, halogen, substituted or unsubstituted (C1-C30)alkyl, substituted or unsubstituted (C6-C30)aryl, substituted or unsubstituted (C3-C30)heteroaryl, NR₇₁R₇₂, and R₇₉R₈₀R₈₁Si-,
the R₇₁ through R₇₂ independently represent substituted or unsubstituted (C1-C30)alkyl, substituted or unsubstituted (C6-C30)aryl or substituted or unsubstituted (C3-C30)heteroaryl, and the R₇₉ through R₈₁ independently represent substituted or unsubstituted (C1-C30)alkyl or substituted or unsubstituted (C6-C30)aryl, and e represents an integer of 0 through 2.

6. The organic electroluminescent device of claim 1, wherein Chemical Formula 1 is selected from the following
structures.

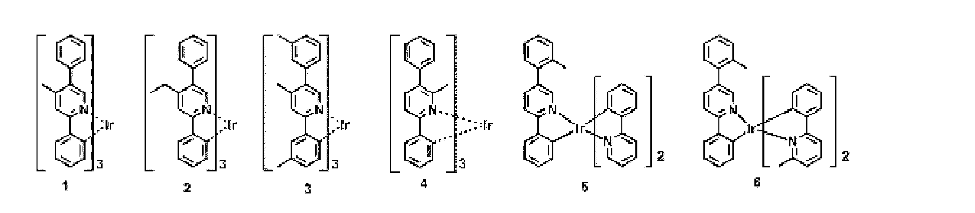

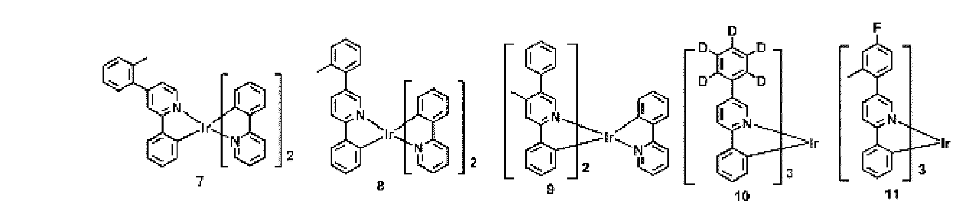

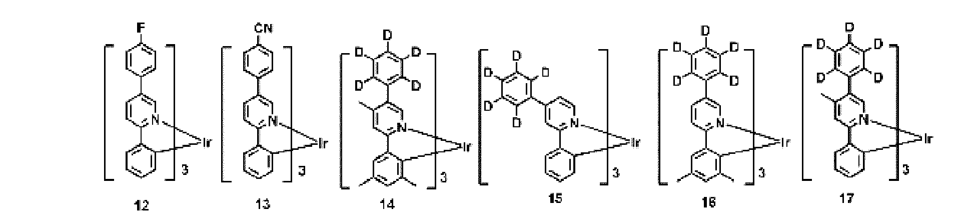

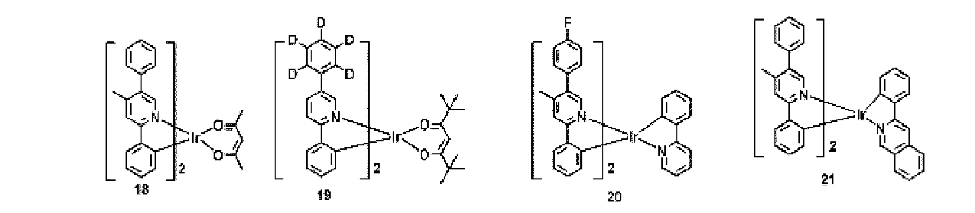

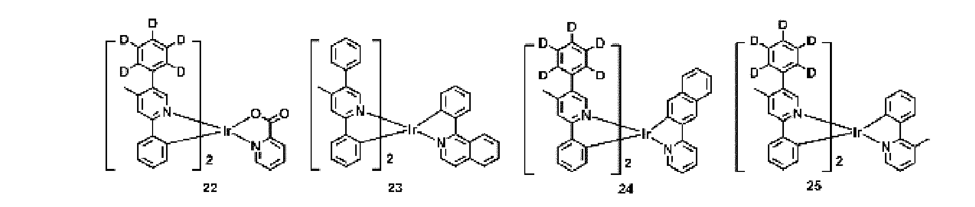

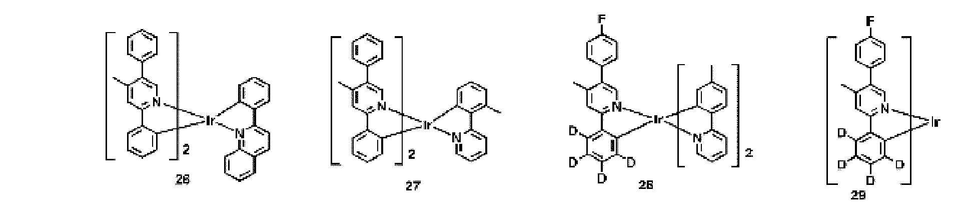

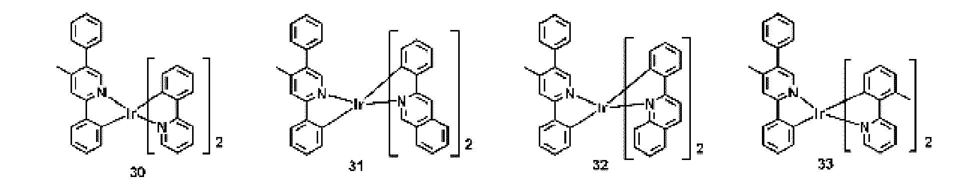

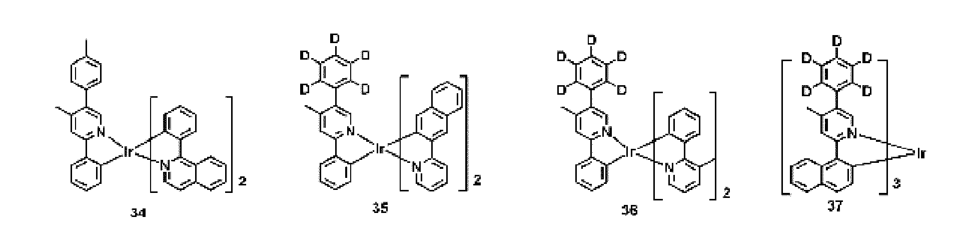

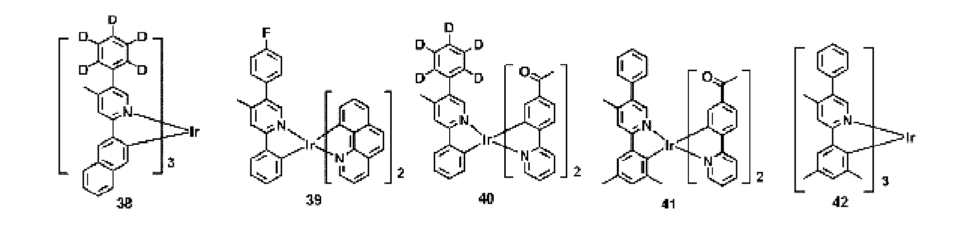

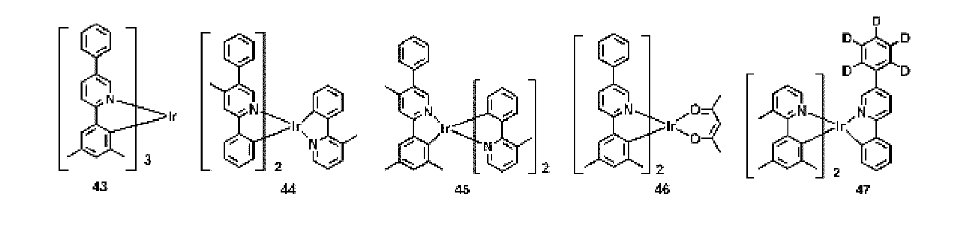

7. The organic electroluminescent device of claim 1, wherein Chemical Formulas 2 to 4 are selected from the following structures.

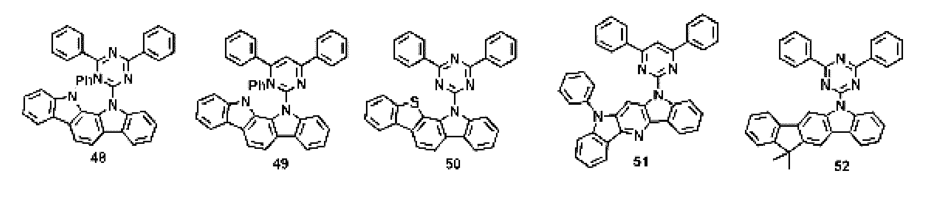

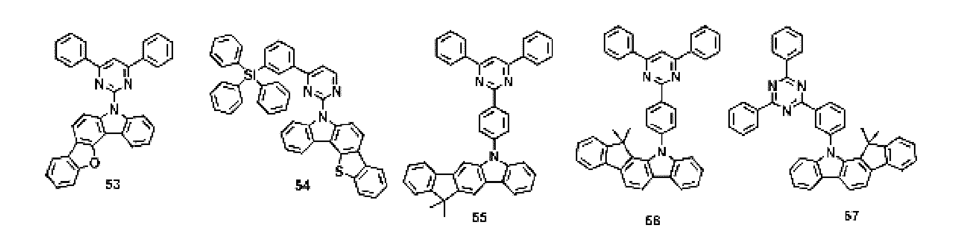

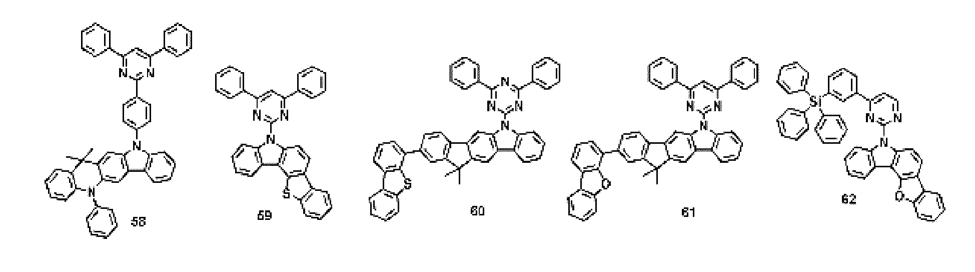

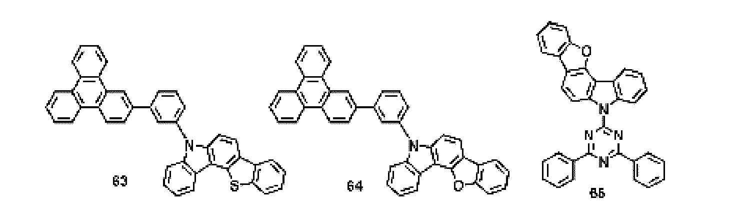

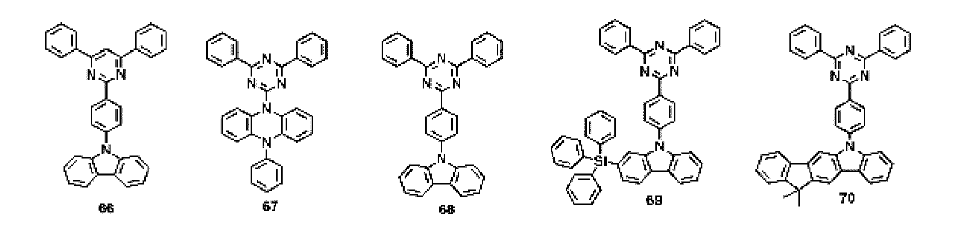

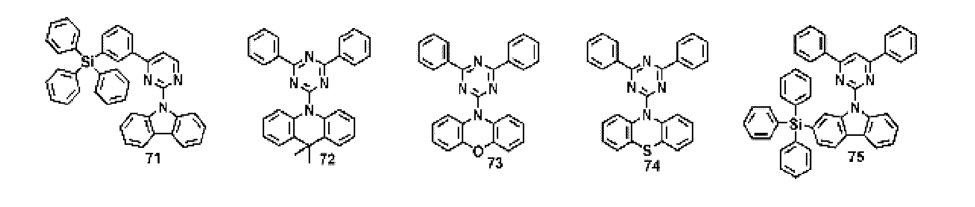

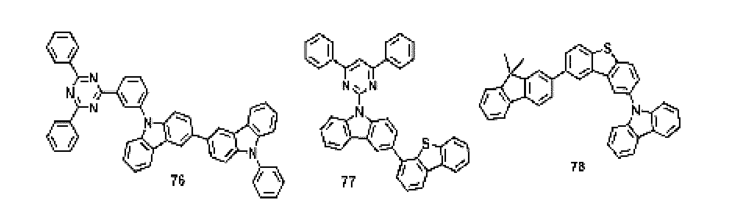

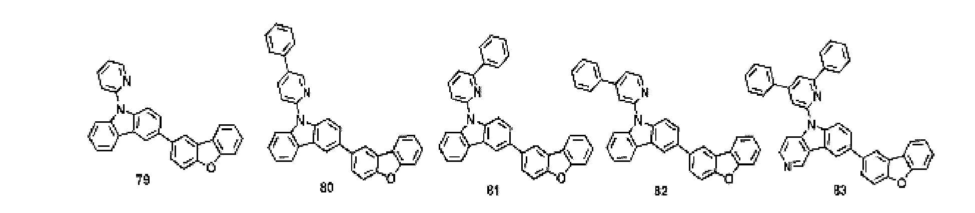

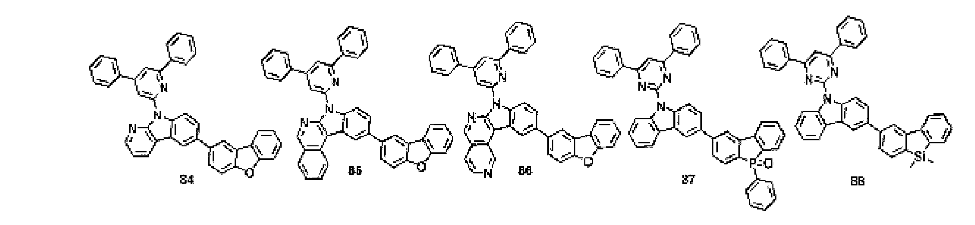

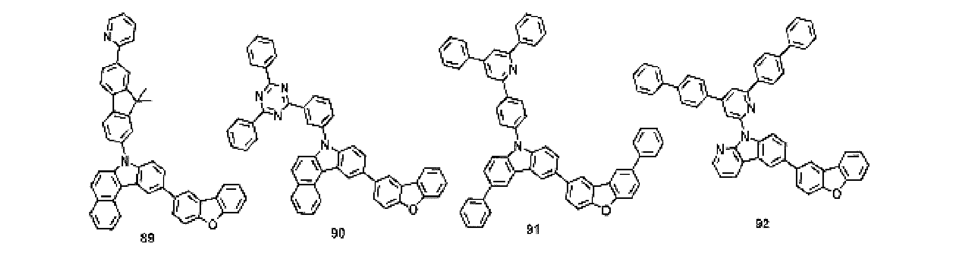

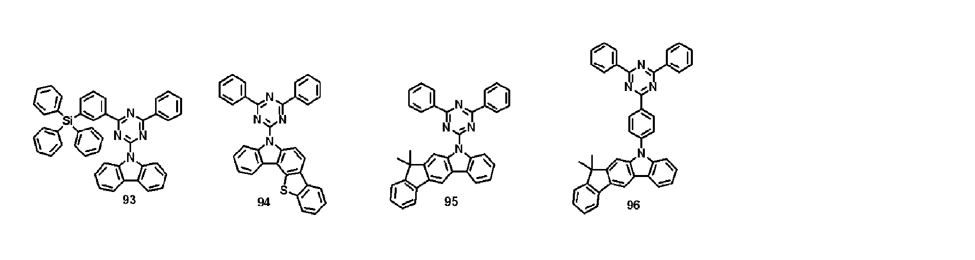

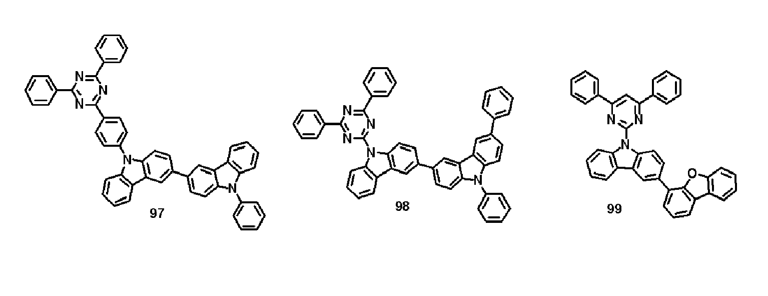

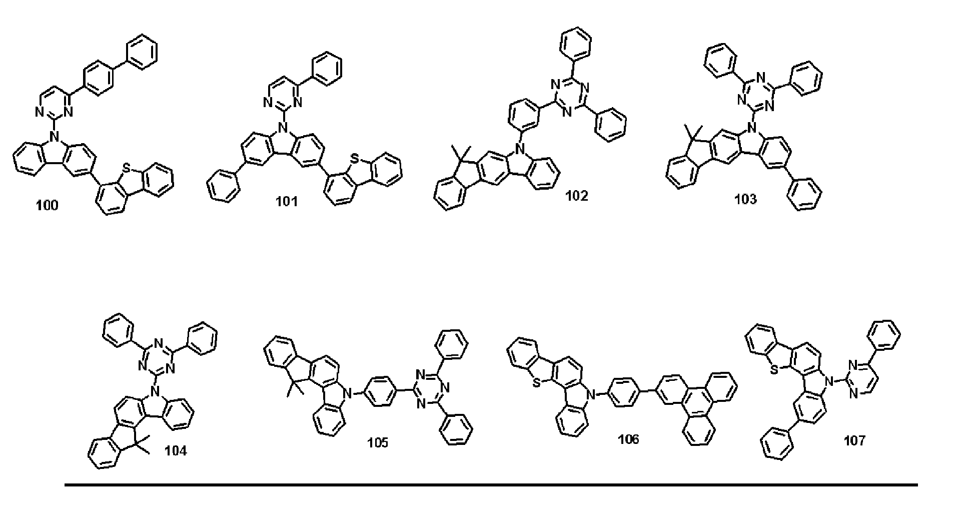

8. The organic electroluminescent device of claim 1, wherein the organic layer comprises an electroluminescent layer and a charge generating layer at the same time.

9. The organic electroluminescent device of claim 1, wherein the organic layer further comprises one or more organic electroluminescent layers emitting red, green or blue light to emit white light.

10. The organic electroluminescent device of claim 1, wherein a doping concentration of the dopant compound based on the host compound in the electroluminescent layer is in a range of below 20 wt%.
